# EUROPEAN PATENT SPECIFICATION

(11) **EP 1 941 843 B1**
(45) Date of publication and mention of the grant of the patent: **17.11.2021**
(21) Application number: 08005936.3
(22) Date of filing: 17.06.2005
(51) Int. Cl.: A61C 13/00, G01J 3/46, G01B 11/25, G01J 3/50, A61B 5/107, A61B 5/00, G01B 11/24, A61B 1/00, A61B 1/06, A61B 1/24, A61B 1/247, A61C 9/00, G01J 3/02

(54) **Method and apparatus for colour imaging a three-dimensional structure**
Verfahren und Vorrichtung zur Farbbildgebung einer dreidimensionalen Struktur
Procédé et appareil d'imagerie couleur d'une structure tridimensionnelle

(30) Priority: 17.06.2004 US 580108 P; 17.06.2004 US 580109 P
(43) Date of publication of application: 09.07.2008
(62) Divisional of application: 05013108.5
(73) Proprietor: Align Technology, Inc., San Jose, CA 95134 (US)
(72) Inventor: Babayoff, Noam, Rishon le Zion 75670 (IL)
(74) Representative: Leach, Sean Adam

(56) References cited:
- WO-A-89/11260
- WO-A2-03/105289
- DE-A1- 19 636 354
- US-A- 4 575 805
- US-A- 4 935 635
- US-A- 5 749 830
- US-A1- 2004 029 068
- US-B1- 6 205 243
- US-B1- 6 208 788
- US-B1- 6 594 539
- US-B1- 6 697 164

## Description

### FIELD OF THE INVENTION

The present invention relates to optical scanners, particularly for providing a digital representation of three-dimensional objects including colour. The invention finds particular application in the surveying of the intraoral cavity.

### BACKGROUND OF THE INVENTION

Many methods have been developed for obtaining the three dimensional location of surface points of an object, for a host of applications including, inter alia, the intraoral cavity. Techniques for direct non-contact optical measurement, in particular for direct optical measurement of teeth and the subsequent automatic manufacture of dentures, are known. The term "direct optical measurement" signifies surveying of teeth in the oral cavity of a patient. This facilitates the obtainment of digital constructional data necessary for the computer-assisted design (CAD) or computer-assisted manufacture (CAM) of tooth replacements without having to make any cast impressions of the teeth. Such systems typically include an optical probe coupled to an optical pick-up or receiver such as charge coupled device (CCD) and a processor implementing a suitable image processing technique to design and fabricate virtually the desired product. Such methods include, for example, confocal imaging techniques as described in WO 00/08415 assigned to the present assignee. These methods provide a digital three-dimensional surface model that is inherently monochromatic, i.e., no colour information is obtained in the imaging process.

Associating colour information with three-dimensional objects is not straightforward, particularly when the position information is obtained by using a three dimensional scanning method, while the colour information is obtained by using a two dimensional scanning method. The problem of conformally mapping the two dimensional colour information onto the three dimensional surface model is difficult and it is common for mismatching of the colour with three-dimensional points to occur. Essentially, where two-dimensional colour detectors are used for obtaining the colour information, it is difficult to accurately associate colour information from the detectors with the correct points on the three dimensional surface model, particularly where relative movement between the object and the device occurs between the acquisition of the three-dimensional topological data and acquisition of the two-dimensional image data.

EP 837 659 describes a process and device for obtaining a three dimensional image of teeth. Three-dimensional surface data is obtained by first covering the surface with an opaque, diffusely reflecting material, and the object is illuminated with monochromatic light. The image of the object under the layer is obtained by the process described in US 4,575,805 using intensity pattern techniques. In order to obtain a two-dimensional colour image of the object, the reflecting layer has to be removed. The method thus requires the camera to be manually re-aligned so that the two-dimensional colour image should more or less correspond to the same part of the object as the three dimensional image. Then, the three dimensional image may be viewed on a screen as a two-dimensional image, and it is possible to superimpose on this two-dimensional image the two-dimensional colour image of the teeth taken by the camera.

US 6,594,539 provides an intraoral imaging system that produces images of a dental surface, including three dimensional surface images and also two dimensional colour images, with the same camera.

In 5,440,393, the shape and dimensions of a dental patients mouth cavity including upper and lower tooth areas and the jaw structure, are measured by an optical scanner using an external radiation source, whose reflected signals are received externally and converted into electronic signals for analysis by a computer. Both surface radiation and reflection from translucent internal surfaces are scanned, and processing of reflections may involve a triangulation system or holograms.

In US 5,864,640, a scanner is described having a multiple view detector responsive to a broad spectrum of visible light. The detector is operative to develop several images of a three dimensional object to be scanned. The images are taken from several relative angles with respect to the object. The images depict several surface portions of the object to be scanned. A digital processor, coupled to the detector, is responsive to the images and is operative to develop with a computational unit 3-D coordinate positions and related image information of the surface portions of the object, and provides 3-D surface information that is linked to colour information without need to conformally map 2-D colour data onto 3-D surface.

Of general background interest, US 4,836,674, US 5,690,486, US 6,525,819, EP 0367647 and US 5,766,006 describe devices for measuring the colour of teeth.

US 6,205,243 B1 describes a system and a method for collection of data in construction of a computer model based on a multi-resolution mesh to describe surface contours and colour of an object. In said document, the triangulation principle is used in order to collect the data.

Document US6697164 B1 discloses a probing member for use in an apparatus for determining surface topology of a teeth portion. The document focuses on the use of confocal imaging of the surface by optionally using multiple wavelengths.

### SUMMARY OF THE INVENTION

The invention is defined by the independent system claim 1 and method claim 29. Preferred embodiments are defined by the dependent claims. Any subject-matter falling outside the scope of the claims is provided for information purposes only.

In accordance with the present invention, a device and method for determining the surface topology and associated colour of at least a portion of a three dimensional structure in the intraoral cavity of a patient using confocal imaging and conformal colour mapping of the structure is provided. Preferred non-limiting embodiments of the invention are concerned with the imaging of a three-dimensional topology of a teeth segment, optionally including such where one or more teeth are missing. This may allow the generation of data for subsequent use in design and manufacture of, for example, prosthesis of one or more teeth for incorporation into said teeth segment.

The colour and surface data is provided in a form that is highly manipulable and useful in many applications including prosthesis colour matching and orthodontics, among others.

In the claimed method the determination of the 3D surface topology of a portion of a three-dimensional structure is carried out using a confocal focusing method, comprising:
**(a)** providing an array of incident light beams propagating in an optical path leading through a focusing optics and a probing face; the focusing optics defining one or more focal planes forward said probing face in a position changeable by said optics, each light beam having its focus on one of said one or more focal plane; the beams generating a plurality of illuminated spots on the structure;
**(b)** detecting intensity of returned light beams propagating from each of these spots along an optical path opposite to that of the incident light;
**(c)** repeating steps (a) and (b) a plurality of times, each time changing position of the focal plane relative to the structure; and
**(d)** for each of the illuminated spots, determining a spot-specific position, being the position of the respective focal plane, yielding a maximum measured intensity of a respective returned light beam; and
based on the determined spot-specific positions, generating data representative of the topology of said portion.

The determination of the spot-specific positions in fact amounts to determination of the in-focus distance. The determination of the spot-specific position may be by measuring the intensity per se, or typically is performed by measuring the displacement (S) derivative of the intensity (I) curve (dI/dS) and determining the relative position in which this derivative function indicates a maximum intensity. The term "spot-specific position (SSP)" will be used to denote the relative in-focus position regardless of the manner in which it is determined. It should be understood that the SSP is always a relative position as the absolute position depends on the position of the sensing face. However the generation of the surface topology does not require knowledge of the absolute position, as all dimensions in the cubic field of view are absolute.

The SSP for each illuminated spot will be different for different spots. The position of each spot in an **X-Y** frame of reference is known and by knowing the relative positions of the focal plane needed in order to obtain maximum intensity (namely by determining the SSP), the **Z** or depth coordinate can be associated with each spot and thus by knowing the **X-Y-Z** coordinates of each spot the surface topology can be generated.

In order to determine the **Z** coordinate (namely the SSP) of each illuminated spot the position of the focal plane may be scanned over the entire range of depth or **Z** component possible for the measured surface portion. Alternatively the beams may have components, each of which has a different focal plane. Thus, by independent determination of SSP for the different light components, e.g. 2 or 3 with respective corresponding 2 or 3 focal planes, the position of the focal planes may be changed by the focusing optics to scan only part of the possible depth range, with all focal planes together covering the expected depth range. Alternatively, the determination of the SSP may involve a focal plane scan of only part of the potential depth range and for illuminated spots where a maximum illuminated intensity was not reached, the SSP is determined by extrapolation from the measured values or other mathematical signal processing methods. Thus, in each case, a **Z**-value is obtained for each point along an **X-Y** grid representing a plurality of light beams. In this manner, a three-dimensional (3D) numerical entity E may be crated, comprising a plurality of coordinates (X, Y, Z) representative of the surface topology of the object being scanned.

According to the present invention, a two dimensional (2D) colour image of the 3D structure that is being scanned is also obtained, but typically within a short time interval with respect to the 3D scan. Further, the 2D colour image is taken at substantially the same angle and orientation with respect to the structure as was the case when the 3D scan was taken. Accordingly, there is very little or no substantial distortion between the **X-Y** plane of 3D scan, and the plane of the image, i.e., both planes are substantially parallel, and moreover substantially the same portion of the structure should be comprised in both the 3D scan and the 2D image. This means that each **X-Y** point on the 2D image substantially corresponds to a similar point on the 3D scan having the same relative **X-Y** values. Accordingly, the same point of the structure being scanned has substantially the same **X-Y** coordinates in both the 2D image and the 3D scan, and thus the colour value at each **X, Y** coordinate of the 2D colour scan may be mapped directly to the spatial coordinates in the 3D scan having the same **X, Y** coordinates, wherein to create a numerical entity I representing the colour and surface topology of the structure being scanned.

Where the **X,Y** coordinates of the colour image do not precisely correspond to those of the 3D scan, for example as may arise where one CCD is for the 3D scanning, while another CCD is used for the 2D colour image, suitable interpolation methods that are not part of the invention may be employed to map the colour data to the 3D spatial data.

The present invention thus provides a device and method for obtaining a numerical entity that represents the colour and surface topology of a three dimensional structure in the intraoral cavity of a patient. The device of the invention provides advantages over monochrome 3D scaners, including such scanners that are based on confocal focusing techniques. For example, the 2D colour image capability on its own enables the dental practitioner to identify the area of interest within the oral cavity with a great degree of confidence in order to better aim the device for the 3D scanning. In other words, an improved viewfinder is automatically provided. Further, rendition of a full colour 3D image of the target area can help the practitioner to decide on the spot whether the scan is sufficiently good, or whether there are still parts of the teeth or soft tissues that should have been included, and thus help the practitioner to deciode whether or not to acquire another 3D colour entity.

Creation of a colour 3D entity that is manipulable by a computer is extremely useful in enabling the practictioner to obtain data from such an entity that is useful for procedures carried out in the dental cavity.

Thus, according to the present invention, a device is provided for determining the surface topology and associated colour of at least a portion of a three dimensional structure, in the intraoral cavity of a patient comprising:
scanning means adapted for providing depth data of said portion corresponding to a two-dimensional reference array substantially orthogonal to a depth direction;
imaging means adapted for providing two-dimensional colour image data of said portion associated with said reference array;
wherein the device is adapted for maintaining a spatial disposition with respect to said portion that is substantially fixed during operation of said scanning means and said imaging means. In other words, operation of the scanning means and the imaging means is substantially or effectively simultaneous in practical terms, and thus the actual time interval that may exist between operation of the two means is so short that the amplitude of any mechanical vibration of the device or movement of the oral cavity will be so small as can be ignored.

The device is adapted for providing a time interval between acquisition of said depth data and acquisition of said colour image data such that substantially no significant relative movement between said device and said portion occurs. The time interval may be between about 0 seconds to about 100 milliseconds, for example 5, 10, 20, 30, 40, 50, 60, 70, 80, 90 or 100 milliseconds, and preferably between about 0 to about 50 milliseconds, and more preferably between about 0 and 20 milliseconds.

The device further comprise processing means for associating said colour data with said depth data for corresponding data points of said reference array. In described embodiments, the operation of said scanning means is based on confocal imaging techniques. Such scanning means comprises:
a probing member with a sensing face;
first illumination means for providing a first array of incident light beams transmitted towards the structure along an optical path through said probing unit to generate illuminated spots on said portion along said depth direction, wherein said first array is defined within said reference array;
a light focusing optics defining one or more focal planes forward said probing face at a position changeable by said optics, each light beam having its focus on one of said one or more focal plane;
a translation mechanism for displacing said focal plane relative to the structure along an axis defmed by the propagation of the incident light beams;
a first detector having an array of sensing elements for measuring intensity of each of a plurality of light beams returning from said spots propagating through an optical path opposite to that of the incident light beams;
a processor coupled to said detector for determining for each light beam a spot-specific position, being the position of the respective focal plane of said one or more focal planes yielding maximum measured intensity of the returned light beam, and based on the determined spot-specific positions, generating data representative of the topology of said portion.

The first array is arranged to provide depth data at a plurality of predetermined spatial coordinates substantially corresponding to the spatial disposition of said incident light beams.

The first illumination means comprises a source emitting a parent light beam and a beam splitter for splitting the parent beam into said array of incident light beams. The first illumination means may comprise a grating or microlens array.

The device may comprise a polarizer for polarizing said incident light beams are polarized. Further, the device may comprise a polarization filter for filtering out from the returned light beams light components having the polarization of the incident light beams.

The illumination unit may comprise at least two light sources and each of said incident beams is composed of light components from the at least two light sources. The at least two light sources emit each a light component of different wavelength. The light directing optics defines a different focal plane for each light component and the detector independently detects intensity of each light components.

The at least two light sources may be located so as to define optical paths of different lengths for the incident light beams emitted by each of the at least two light sources.

Typically, the focusing optics operates in a telecentric confocal mode. Optionally, the light directing optics comprises optical fibers.

Typically, the sensing elements are an array of charge coupled devices (CCD). The detector unit may comprise a pinhole array, each pinhole corresponding to one of the CCDs in the CCD array.

The operation of said imaging means may be based on:
illuminating said portion with three differently- coloured illumination radiations, the said illuminations being combinable to provide white light,
capturing a monochromatic image of said portion corresponding to each said illuminating radiation, and
combining the monochromatic images to create a full colour image,
wherein each said illuminating radiation is provided in the form of a second array of incident light beams transmitted towards the portion along an optical path through said probing unit to generate illuminated spots on said portion along said depth direction, wherein said second array is defined within said reference frame.

The second array is arranged to provide colour data at a plurality of spatial coordinates substantially corresponding to the spatial coordinates of said first array. The device comprises colour illumination means adapted for providing three second illuminating radiations, each of a different colour. The colour illumination means comprises second illumination means for providing said three second illuminating radiations, each of a different colour. Alternatively, the colour illumination means comprises second illumination means for providing two said second illuminating radiations, and wherein said first illumination means provides another said second illuminating radiation each said second illuminating radiation being of a different colour. Optionally, each one of said second illumination radiations is a different one of red, green or blue light. The second illumination means may comprise radiation transmission elements that are configured to be located out of the path of said light beams or said returned light beam at least within said light focusing optics. The probing member may be made from a light transmissive material having an upstream optical interface with said light focusing optics and a reflective face for reflecting light between said optical interface and said sensing face. The second illumination means may be optically coupled to said optical interface for selectively transmitting illuminating radiations in at least two colours to said portion via said sensing face. The colour illumination means may comprise second illumination means for providing two said second illuminating radiations, and wherein said first illumination means provides another said second illuminating radiation each said second illuminating radiation being of a different colour. The probing member may comprise a removable sheath having an inner surface substantially complementary to an outer surface of said probing member, and having a window in registry with said sensing face, wherein said sheath is made from a waveguiding material and is adapted to transmit said light from said second illuminating means from an upstream face thereof to a downstream face associated with said window. The second illumination means may be optically coupled to said upstream face for selectively transmitting said second illuminating radiations in at least two colours to said portion via said downstream face. Preferably, the sheath is disposable after use with a patient.

In another embodiment, the reflective face comprises a dichroic coating, having relatively high reflectivity and low optical transmission properties for a said second illuminating radiation provided by said first illumination means, and relatively low reflectivity and high optical transmission properties for the two said second illuminating radiations provided by said second illumination means.

The second illumination means are adapted for providing second illuminating radiations within said light focusing optics. In particular, the second illumination means may be adapted for providing second illuminating radiations at an aperture stop plane of said light focusing optics.

Optionally, the device further comprises:
a first polarizing element located just downstream of said illumination means so as to polarize the light emitted therefrom;
a second polarizing element located just upstream of said first detector, wherein said second polarizing element is crossed with respect to the first polarizing element; and
a quarter waveplate at the downstream end of said device.

Further optionally the second illumination means are adapted for selective movement in the depth direction.

The device may comprise a mirror inclined to the optical axis of said light focusing optics and having an aperture configured to allow said light beams and said returning light beams to pass therethrough without being optically affected by said mirror, and wherein said second illumination means comprises at least one white illumination source optically coupled with suitable colour filters, said filters selectively providing illumination radiation in each colour in cooperation with said white illumination source, wherein said mirror is coupled to said white illumination source to direct radiation therefrom along said optical axis. The white illumination source may comprise a phosphorus InGaN LED. The filters may be arranged on sectors of a rotatable disc coupled to a motor, predetermined selective angular motion of said disc selectively couples said white illumination source to each said filter in turn.

Optionally, the second illumination means are in the form of suitable LED's, comprising at least one LED for providing illumination radiation in each colour. Optionally, the second illumination means are in the form of suitable LED's, comprising at least one white illumination source optically coupled with suitable colour filters, said filters selectively providing illumination radiation in each colour in cooperation with said white illumination source. The white illumination source may comprise a phosphorus InGaN LED. The filters may be arranged on sectors of a rotatable disc coupled to a motor, predetermined selective angular motion of said disc selectively couples said white illumination source to each said filter in turn. The device may further comprise a plurality of optical fibers in optical communication with said filters and with radiation transmission elements comprised in said second illumination means.

The first detector is adapted for selectively measuring intensity of each said second illuminating radiation after reflection from said portion.

Alternatively, the operation of said imaging means is based on illuminating said portion with substantially white illumination radiation, and capturing a colour image of said portion, wherein said white illuminating radiation is provided in the form of a second array of incident light beams transmitted towards the portion along an optical path through said probing unit to generate illuminated spots on said portion along said depth direction, wherein said second array is defined within said reference frame. The second array is arranged to provide colour data at a plurality of spatial coordinates substantially corresponding to the spatial coordinates of said first array. The imaging means comprises:-
white illumination radiation means;
second detector having an array of sensing elements for measuring intensity of said white illuminating radiation after reflection from said portion.

Alternatively, the operation of said imaging means is based on illuminating said portion with substantially white illumination radiation, selectively passing radiation reflected from said portion through a number of colour filters, capturing a monochromatic image of said portion corresponding to each said filter, and combining the monochromatic images to create a full colour image, wherein said illuminating radiation is provided in the form of a second array of incident light beams transmitted towards the portion along an optical path through said probing unit to generate illuminated spots on said portion along said depth direction, wherein said second array is defined within said reference frame. The second array is arranged to provide colour data at a plurality of spatial coordinates substantially corresponding to the spatial coordinates of said first array.

Alternatively, the operation of said imaging means is based on illuminating said portion with three differently- coloured illumination radiations, capturing a monochromatic image of said portion corresponding to each said illuminating radiation, and combining the monochromatic images to create a full colour image, wherein each said illuminating radiation is provided in the form of a second array of incident light beams transmitted towards the portion along an optical path through said probing unit to generate illuminated spots on said portion along said depth direction, wherein said second array is defined within said reference frame, and wherein said illuminating radiations are provided by said first illumination source. The second array is arranged to provide colour data at a plurality of spatial coordinates substantially corresponding to the spatial coordinates of said first array.

The device may further comprise a tri- colour sequence generator for controlling the illumination of said portion with said second illuminating radiations.

The device further comprises a processor coupled to said detector for conformally mapping colour data provided by said imaging means to said depth data provided by said scanning means for each said spatial coordinates of said first array to provide a colour three-dimensional numerical entity comprising a plurality of data points, each data point comprising three-dimensional surface coordinate data and colour data associated therewith. The device may also optionally comprise a unit for generating manufacturing data for transmission to CAD/CAM device based on said entity, and a communication port of a communication medium.

The device is adapted for determining colour and surface topology of a teeth portion, but may be used for determining colour and surface topology of any suitable surface in the intraoral cavity of a patient.

The present invention is also directed to a method for determining the surface topology and associated colour of at least a portion of a three dimensional structure, in the intraoral cavity of a patient comprising:
(a) providing depth data of said portion corresponding to a two-dimensional reference array substantially orthogonal to a depth direction;
(b) providing two-dimensional colour image data of said portion associated with said reference array;
(c) ensuring that a spatial disposition with respect to said portion during steps (a) and (b) is substantially fixed;
(d) conformally mapping said colour data to said depth data for said reference array.

Preferably, in step (c), a minimum time interval is allowed between acquisition of said depth data and acquisition of said image data. The time interval may be between about 0 seconds to about 100 milliseconds, preferably between 0 and 50 milliseconds, and more preferably between 0 and 20 milliseconds.

In described embodiments, the depth data is provided using confocal imaging techniques. The method then comprises:
(i) providing a first array of incident light beams defined within said reference array propagating in an optical path leading through a focusing optics and through a probing face; the focusing optics defining one or more focal planes forward said probing face in a position changeable by said optics, each light beam having its focus on one of said one or more focal plane; the beams generating a plurality of illuminated spots on the structure;
(ii) detecting intensity of returned light beams propagating from each of these spots along an optical path opposite to that of the incident light;
(iii) repeating steps (i) and (ii) a plurality of times, each time changing position of the focal plane relative to the structure;
(iv) for each of the illuminated spots, determining a spot-specific position, being the position of the respective focal plane yielding a maximum measured intensity of a respective returned light beam; and
(v) generating data representative of the topology of said portion.

Step (ii) may be based on illuminating said portion with at least three differently- coloured illumination radiations, said illumination radiations being combinable to produce white radiation, capturing a monochromatic image of said portion corresponding to each said illuminating radiation, and combining the monochromatic images to create a full colour image, wherein each said illuminating radiation is provided in the form of a second array of incident light beams transmitted towards the portion along an optical path through said probing unit to generate illuminated spots on said portion along said depth direction, wherein said second array is defined within said reference frame. The second array is arranged to provide colour data at a plurality of spatial coordinates substantially corresponding to the spatial coordinates of said first array.

Optionally, the sources for the at least three coloured illuminations may be located at the confocal system aperture stop, and facing the objective lens of the system. Preferably, the illumination sources are configured to have a relatively low numerical aperture compared with that of the first array of light beams.

Further preferably, the said sources for the coloured illuminations are moveable in the depth direction.

Optionally, the method of the invention further comprises the steps of:
polarizing the emitted coloured illuminations by means of a first polarizing element;
modifying the said polarized colour illuminations on the way to the structure and on their return therefrom by means of a quarter waveplate;
causing the returning colour illuminations to pass through a second polarizing element located just upstream of said first detector, wherein said second polarizing element is crossed with respect to the first polarizing element.

Step (ii) may be based on illuminating said portion with substantially white illumination radiation, selectively passing radiation reflected from said portion through a number of colour filters, capturing a monochromatic image of said portion corresponding to each said filter, and combining the monochromatic images to create a full colour image, wherein said illuminating radiation is provided in the form of a second array of incident light beams transmitted towards the portion along an optical path through said probing unit to generate illuminated spots on said portion along said depth direction, wherein said second array is defined within said reference frame. The second array is arranged to provide colour data at a plurality of spatial coordinates substantially corresponding to the spatial coordinates of said first array.

Step (ii) may be based on illuminating said portion with three differently-coloured illumination radiations, capturing a monochromatic image of said portion corresponding to each said illuminating radiation, and combining the monochromatic images to create a full colour image, wherein each said illuminating radiation is provided in the form of a second array of incident light beams transmitted towards the portion along an optical path through said probing unit to generate illuminated spots on said portion along said depth direction, wherein said second array is defined within said reference frame, and wherein said illuminating radiations are provided by said first illumination source. The second array is arranged to provide colour data at a plurality of spatial coordinates substantially corresponding to the spatial coordinates of said first array.

The data representative of said topology may be used for constructing an object to be fitted within said structure, in the intraoral cavity of a patient, or may be converted into a form transmissible through a communication medium to recipient. Typically, the structure is a teeth segment. The structure may be a teeth segment with at least one missing tooth or a portion of a tooth and said object is said at least one missing tooth or the portion of the tooth. Thus, for example, steps (i) to (v) may be repeated for two different surfaces of said structure to provide surface topologies thereof, and the surface topologies may then be combined to obtain colour and topological data representative of said structure.

### BRIEF DESCRIPTION OF THE DRAWINGS

In order to understand the invention and to see how it may be carried out in practice, a number of embodiments will now be described, by way of non-limiting example only, with reference to the accompanying drawings, in which:
**Fig. 1** illustrates the main elements of preferred embodiments of the invention.
**Figs. 2A, 2B****,** **2C** graphically illustrates the creation of a three dimensional colour entity from a three dimensional monochrome entity and a two dimensional colour entity.
**Fig. 3** graphically illustrates an alignment procedure according to the invention for aligning the X-Y coordinates of a three dimensional monochrome entity with corresponding coordinates of a two dimensional colour entity.
**Figs. 4A** **and** **4B** schematically illustrate the main elements of a portion of the invention used for providing a three dimensional monochrome entity.
**Figs. 5A, 5B, 5C** illustrate in plan view, side view and isometric view, respectively, a probe used in first embodiment of the invention to provide a two dimensional colour entity.
**Fig. 6** illustrates in side view a sheath for a probe used in second embodiment of the invention to provide a two dimensional colour entity.
**Fig. 7A** illustrates in side view a probe used in third embodiment of the invention to provide a two dimensional colour entity. **Fig. 7B** illustrates the transmission and reflection characteristics of a typical dichroic coating used in the probe of Fig. 7A.
**Fig. 8** illustrates in side view the general arrangement of the main elements used in fourth embodiment of the invention to provide a two dimensional colour entity.
**Fig. 9** illustrates an LED arrangement which may be used with the embodiment of Fig. 8.
**Fig. 10** illustrates an alternative illumination arrangement used with the embodiment of Figure 8. Fig. 10A illustrates details of the tri- colour disc used with the illumination arrangement of Fig. 10.
**Fig. 11** illustrates in side view the general arrangement of the main elements used in fifth embodiment of the invention to provide a two dimensional colour entity.
**Fig. 12** illustrates in side view the general arrangement of the main elements used in sixth embodiment of the invention to provide a two dimensional colour entity.
**Fig. 13** illustrates in side view the general arrangement of the main elements used in seventh embodiment of the invention to provide a two dimensional colour entity.

### DETAILED DESCRIPTION

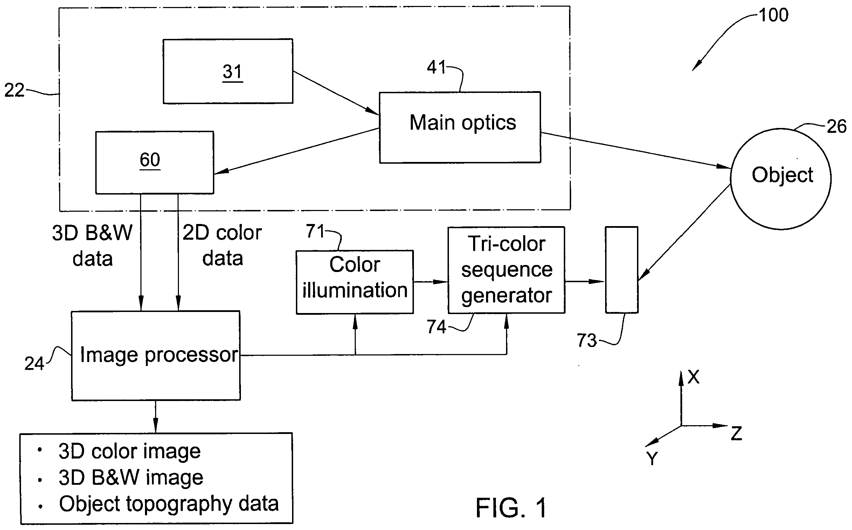

Reference is first being made to Fig. 1 which illustrates the general relationship between the various elements of the device of the invention, generally designated with the numeral **100,** according to the embodiments described herein.

The device **100** comprises a main illumination source **31** for illuminating the object of interest 26 in the intraoral cavity of a patient, and is optically coupled to main optics **41** to provide depth **Z** values for an array range of **X-Y** points (according to a known frame of reference) along the surface of the object **26.** Detection optics **60** comprises an image sensor, typically a CCD, that is preferably monochromatic to maximise the resolution of the device, and which typically defines the X-Y frame of reference. Alternatively, the CCD may be adapted to receive colour images. The detection optics **60** receives image data from the main optics **41** and the image processor **24** determines the depth **Z** values for each **X-Y** point illuminated on the object **26** based on this image data. In this manner, a manipulable three-dimensional numerical entity **E** comprising the surface coordinates of the object 26 of the intraoral cavity of a patient.

The device **100** further comprises colour illuminating means, such as for example a tri- colour sequence generator **74,** for selectively illuminating the object **26** with suitable colours, typically Green, Red and Blue, and for each such monochromatic illumination, a two dimensional image of the object **26** is captured by the detection optics **60.** The processor **24** then processes the three differently coloured monochromatic images and combines the same to provide a full colour 2D image of the object. The device **100** is configured for providing colour data for an array of X-Y points that is according to the same frame of reference as the X-Y array used for obtaining the 3D entity.

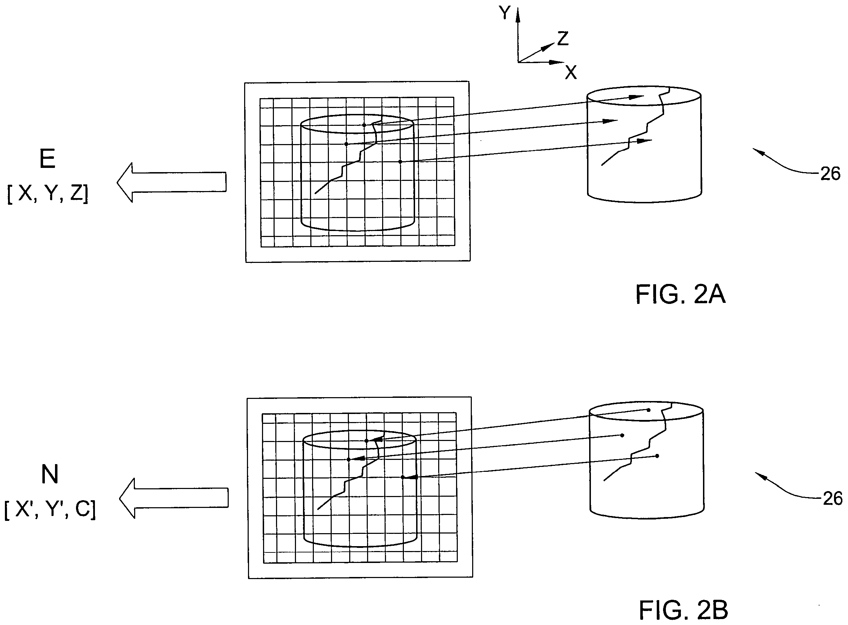

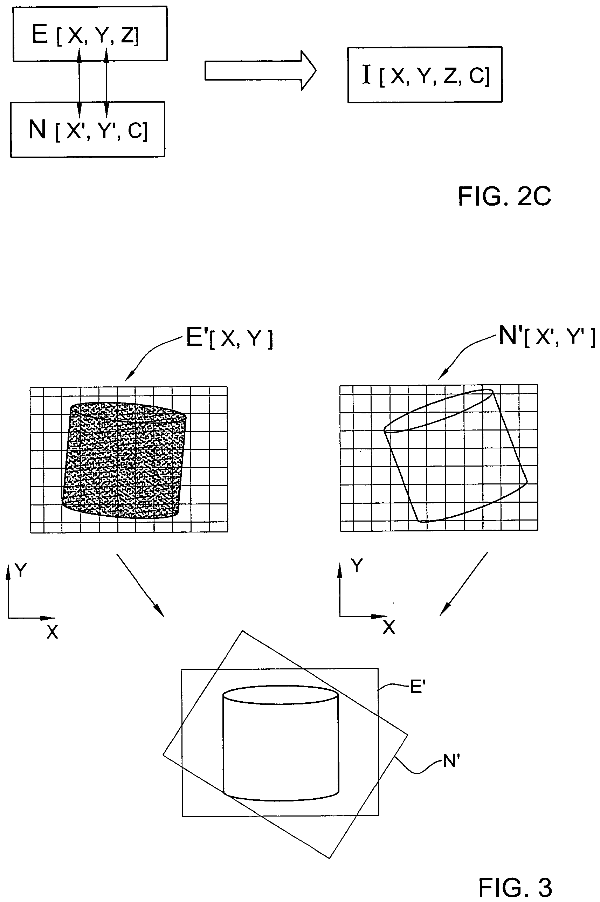

The processor **24** aligns the 2D colour image with the 3D entity previously created, and then provides colour values to this entity by mapping colour values to the entity at aligned **X-Y** points. Such alignment is straightforward because both the 3D data and the 2D colour data are treferenced to the same X-Y frame of reference. Referring to Figs. 2A, 2B, 2C, the mapping procedure is performed as follows. A three-dimensional numerical entity **E** is obtained by determining depth **Z**-values for a grid of **X-Y** points, illuminated via main optics **41** and determined by image processor **24.** The entity **E** thus comprises an array of **(X, Y, Z)** points, as illustrated in Fig. 2A. The **X-Y** plane of entity **E** is substantially parallel to the sensing face of the image sensing means of the detection optics **60,** typically a CCD. Almost concurrently, i.e., either just before or just after the readings for determining the 3D entity **E** are obtained by the detection optics **60,** a 2D colour image of the object **26** is taken using the same detection optics **60,** at substantially the same relative spatial disposition between the detection optics **60** and the object **26,** Fig. 2B. If a monochromatic CCD is used, the 2D colour image obtained is a composite created from three separate monochromatic images, each provided by illuminating the object **26** with a different colour, such as for example red, green and blue. The 2D colour image thus corresponds to another entity **N** comprised of the location and colour value of each pixel forming this image, **(X', Y', C).** The **X'-Y'** coordinates of the pixels are on a plane substantially parallel to the **X-Y** plane of the entity **E,** and furthermore these coordinates represent substantially the same part of the object **26** as the **X-Y** coordinates of entity **E.** The reason for this is that the optical information that is used for creating both the 3D entity **E** and the colour 2D entity **N** are obtained almost simultaneously with a very small time interval therebetween, and typically there is insufficient time for any significant relative movement between the image plane of the detection optics **60** and the object **26** to have occurred between the two scans. Thus, similar **X-Y** and **X'-Y'** coordinates in the entities **E** and **N,** resepectively, will substantially represent the same part of the object **26.** Accordingly, the colour value **C** of each pixel of entity **N** can be mapped to the data point of entity **E** having **X-Y** coordinates that are the same as the **X'-Y'** coordinates of the pixel, whereby to create another entity **I** comprising surface coordinate and colour data, **(X, Y, Z, C),** as illustrated in Fig. 2C.

Were the relative angle and disposition between the plane of the sensing face of the detection optics **60** with respect to the object **26** change significantly between the 2D and the 3D scans, then the **X-Y** coordinates of entity **E** having similar values to the **X'-Y'** coordinates of entity **N** could correspond to different parts of the object **26,** and thus it may then be difficult to map the colour values of entity **N** to entity **E.** However, if only a small movement between the detection optics **60** with respect to the object **26** occurs, particularly involving a relative translation or a rotation about the depth direction (Z), but substantially no change in the angular disposition between detection optics **60** and the object **26** about the **X** or **Y** axes, it may still be posible to map the colour values of entity **N** to entity **E,** but first an alignment procedure must be followed.

Referring to Fig. 3, such an alignment procedure may be based on optical character recognition (OCR) techniques that are not part of the present invention.

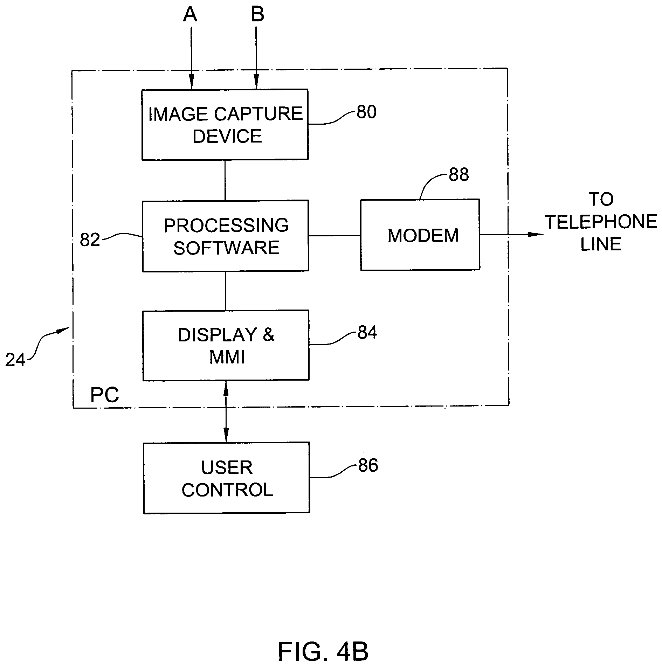

In the **X-Y** plane, the **X-Y** coordinates of entity **E** can be divided up into two groups, one group comprising **Z** values corresponding to the depth of the object, and a second group for which no reasonable **Z** value was found, and this group corresponds to the background relative to object **26.** The profiles of shapes represented by the **X-Y** coordinates of the first group of entity **E,** herein referred to as another entity **E',** are then optically compared with profiles of shapes corresponding to the **X'-Y'** coordinates of entity **N,** herein referred to as another entity **N'.** Accordingly, entity **E'** is translated or rotated (coplanarly) with respect to entity **N'** until a best fit between the optical shapes between the two entities is obtained, using OCR techniques that are well known in the art. Typically, the image processor, or another computer, will attempt to align the outer border of the object **26** as seen along the **Z**-axis and encoded in entity **E** with optical elements in the 2D colour image encoded in entity **N.** Thereafter, the colour value **C** of each **X'-Y'** coordinate of entity **N** is mapped to the appropriate data point of entity **E** having the aligned **X-Y** coordinates corresponding thereto. The colour mapping operation to create entity I may be executed by any suitable microprocessor means, typically processor **24** of the device **100** (Fig. 4B).

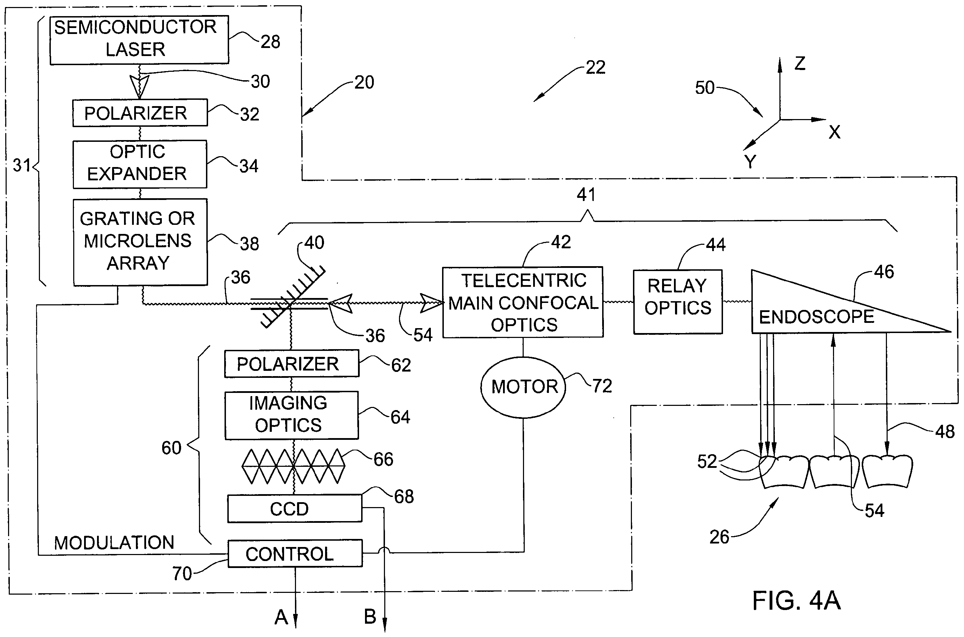

The main optics **41,** main illumination source **31,** detection optics **60** and image processor **24** are now described with reference to Figs. 4A and 4B which illustrate, by way of a block diagram an embodiment of a system **20** for confocal imaging of a three dimensional structure according to WO 00/08415 assigned to the present assignee.

The system **20** comprises an optical device **22** coupled to a processor **24.** Optical device **22** comprises, in this specific embodiment, a semiconductor laser unit **28** emitting a laser light, as represented by arrow **30.** The light passes through a polarizer **32** which gives rise to a certain polarization of the light passing through polarizer **32.** The light then enters into an optic expander **34** which improves the numerical aperture of the light beam **30.** The light beam **30** then passes through a module **38,** which may, for example, be a grating or a micro lens array which splits the parent beam **30** into a plurality of incident light beams **36,** represented here, for ease of illustration, by a single line. The operation principles of module **38** are known per se and the art and these principles will thus not be elaborated herein.

The optical device **22** further comprises a partially transparent mirror **40** having a small central aperture. It allows transfer of light from the laser source through the downstream optics, but reflects light travelling in the opposite direction. It should be noted that in principle, rather than a partially transparent mirror other optical components with a similar function may also be used, e.g. a beam splitter. The aperture in the mirror **40** improves the measurement accuracy of the apparatus. As a result of this mirror structure the light beams will yield a light annulus on the illuminated area of the imaged object as long as the area is not in focus; and the annulus will turn into a completely illuminated spot once in focus. This will ensure that a difference between the measured intensity when out-of- and in-focus will be larger. Another advantage of a mirror of this kind, as opposed to a beam splitter, is that in the case of the mirror internal reflections which occur in a beam splitter are avoided, and hence the signal-to-noise ratio improves.

The unit further comprises a confocal optics **42,** typically operating in a telecentric mode, a relay optics **44,** and an endoscopic probing member **46.** Elements **42, 44** and **46** are generally as known per se. It should however be noted that telecentric confocal optics avoids distance-introduced magnification changes and maintains the same magnification of the image over a wide range of distances in the **Z** direction (the **Z** direction being the direction of beam propagation). The relay optics enables to maintain a certain numerical aperture of the beam's propagation.

The endoscopic probing member **46** typically comprises a rigid, light-transmitting medium, which may be a hollow object defining within it a light transmission path or an object made of a light transmitting material, e.g. a glass body or tube. At its end, the endoscopic probe typically comprises a mirror of the kind ensuring a total internal reflection and which thus directs the incident light beams towards the teeth segment **26.** The endoscope **46** thus emits a plurality of incident light beams **48** impinging on to the surface of the teeth section.

Incident light beams **48** form an array of light beams arranged in an **X-Y** plane, in the Cartesian frame **50,** propagating along the **Z** axis. As the surface on which the incident light beams hits is an uneven surface, the illuminated spots **52** are displaced from one another along the **Z** axis, at different (Xᵢ,Yᵢ) locations. Thus, while a spot at one location may be in focus of the optical element **42,** spots at other locations may be out-of-focus. Therefore, the light intensity of the returned light beams (see below) of the focused spots will be at its peak, while the light intensity at other spots will be off peak. Thus, for each illuminated spot, a plurality of measurements of light intensity are made at different positions along the Z-axis and for each of such (Xᵢ,Yᵢ) location, typically the derivative of the intensity over distance (Z) will be made, the Zᵢ yielding maximum derivative, Z₀, will be the in-focus distance. As pointed out above, where, as a result of use of the punctured mirror **40,** the incident light forms a light disk on the surface when out of focus and a complete light spot only when in focus, the distance derivative will be larger when approaching in-focus position thus increasing accuracy of the measurement.

The light scattered from each of the light spots includes a beam travelling initially in the **Z**-axis along the opposite direction of the optical path traveled by the incident light beams. Each returned light beam **54** corresponds to one of the incident light beams **36.** Given the unsymmetrical properties of mirror **40,** the returned light beams are reflected in the direction of the detection optics **60.** The detection optics **60** comprises a polarizer **62** that has a plane of preferred polarization oriented normal to the plane polarization of polarizer **32.** The returned polarized light beam **54** pass through an imaging optic **64,** typically a lens or a plurality of lenses, and then through a matrix **66** comprising an array of pinholes. CCD camera has a matrix or sensing elements each representing a pixel of the image and each one corresponding to one pinhole in the array **66.**

The CCD camera is connected to the image-capturing module **80** of processor unit **24.** Thus, each light intensity measured in each of the sensing elements of the CCD camera is then grabbed and analyzed, in a manner to be described below, by processor **24.**

Unit **22** further comprises a control module **70** connected to a controlling operation of both semi-conducting laser **28** and a motor **72.** Motor **72** is linked to telecentric confocal optics **42** for changing the relative location of the focal plane of the optics **42** along the Z-axis. In a single sequence of operation, control unit **70** induces motor 72 to displace the optical element **42** to change the focal plane location and then, after receipt of a feedback that the location has changed, control module **70** will induce laser **28** to generate a light pulse. At the same time, it will synchronize image-capturing module **80** to grab data representative of the light intensity from each of the sensing elements. Then in subsequent sequences the focal plane will change in the same manner and the data capturing will continue over a wide focal range of optics **44.**

Image capturing module **80** is connected to a CPU **82,** which then determines the relative intensity in each pixel over the entire range of focal planes of optics **42, 44.** As explained above, once a certain light spot is in focus, the measured intensity will be maximal. Thus, by determining the Zᵢ corresponding to the maximal light intensity or by determining the maximum displacement derivative of the light intensity, for each pixel, the relative position of each light spot along the **Z**-axis can be determined. Thus, data representative of the three-dimensional pattern of a surface in the teeth segment, can be obtained. This three-dimensional representation may be displayed on a display **84** and manipulated for viewing, e.g. viewing from different angles, zooming-in or out, by the user control module **86** (typically a computer keyboard).

The device **100** further comprises means for providing a 2D colour image of the same object 26 of the intraoral cavity of a patient.

A number of techniques suitable for providing the colour image are described below.

The first technique is based on illuminating the object **26** sequentially with three different coloured lights such as red, green and blue, and capturing a monochromatic image corresponding to each colour via CCD **68** and the image capture device **80** (see Figs. 4A, 4B). Referring to Fig. 1, tri- colour light sources **71,** i.e., one or more light sources that provide illuminating radiations to the object **26** in a plurality of different colours, are coupled to a tri- colour sequence generator **74,** which are suitably controlled by the processing unit **24** to provide the three coloured illuminations via delivery optics **73** in a predetermined sequence. The coloured illuminations are provided at a relative short time interval, typically in the range of about 0 to 100 milliseconds, in some cases being in the order of 50 milliseconds or 20 milliseconds, for example, with respect to the 3D scan, directly before or after the same. Suitable processing software **82** combines the three images to provide a 2D colour image comprising an array of data points having location **(X,Y)** and colour **(C)** information for each pixel of the 2D colour image.

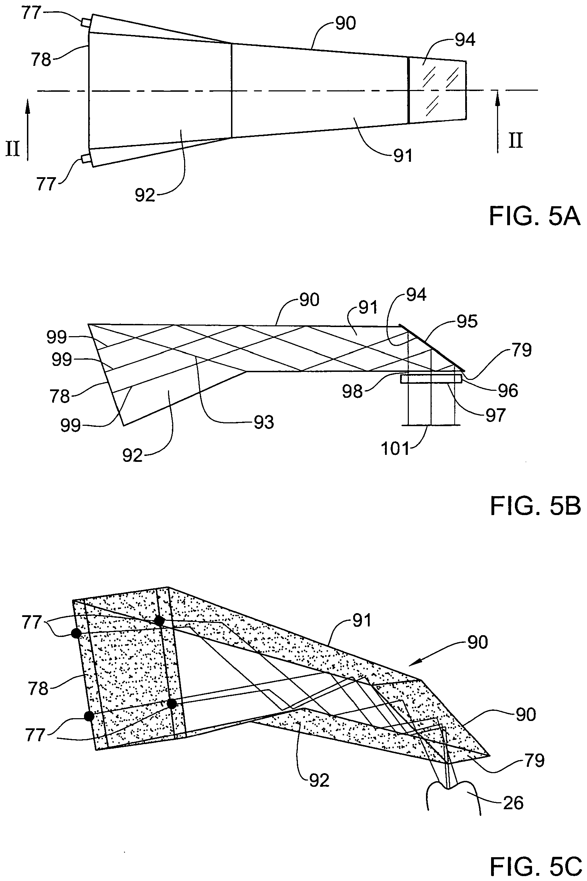

According to a first embodiment of the device **100,** the delivery optics **73** is integral with endoscope **46,** which is in the form of a probing member **90,** as illustrated in Figs. 5A, 5B and 5C. The probing member **90** is made of a light transmissive material, typically glass and is composed of an anterior segment **91** and a posterior segment **92,** tightly glued together in an optically transmissive manner at **93.** Slanted face **94** is covered by a totally reflective mirror layer **95.** Glass disk **96** defining a sensing surface **97** may be disposed at the bottom in a manner leaving an air gap **98.** The disk is fixed in position by a holding structure which is not shown. Three light rays are **99** from the main optics **42** are represented schematically. As can be seen, they bounce at the walls of the probing member at an angle in which the walls are totally reflective and finally bounce on mirror **95** and reflected from there out through the sensing face **97.** The light rays focus on focusing plane **101,** the position of which can be changed by the focusing optics (not shown in this figure). The probe member **90** comprises an interface **78** via which optical communication is established with the relay optics **44** and the remainder of the device **100.** The probe **90** further comprises a plurality of tri- colour LED's **77,** for providing the coloured illumination to the object **26.**

The LED's 77 typically comprise different LED's for providing blue radiation and green radiation when red illuminating radiation is used as the illumination source 31 for the main optics 41 when creating the 3D entity. Alternatively, if a blue illuminating radiation is used as the illumination source 31, the LED's 77 may comprise green and red LED's, and if a green illuminating radiation is used as the illumination source 31, LED's 77 may comprise blue and red LED's.

The tri- colour LED's **77** are each capable of providing an illumination radiation in one of three colours, typically red, green or blue, as controlled via the tri- colour sequence generator. Alternatively, a plurality of LED's in three groups, each group providing illumination in one of the desired colours, may be provided. The LED's **77** are located at the periphery of the interface **78** such that the LED's do not interfere with the other optical operations of the device 100. In particular such operations include the transmission of the illuminating radiation for the confocal focusing operations, and also the transmission of reflected light from the object **26** to the main optics **41** to provide the 3D entity or the 2D colour entity. The LED's are mounted substantially orthogonally with respect to the interface **78,** and thus, as illustrated in Fig. 5C, light from each of the LED's 77 is transmitted by internal reflection with respect to the walls of the probe **90,** to the user interface end **79** of the probe.

Preferably, the device **100** according to a variation of the first embodiment is further adapted for providing improved precision of the colour data obtained therewith, in a similar manner to that described herein for the fourth embodiment, *mutatis mutandis.*

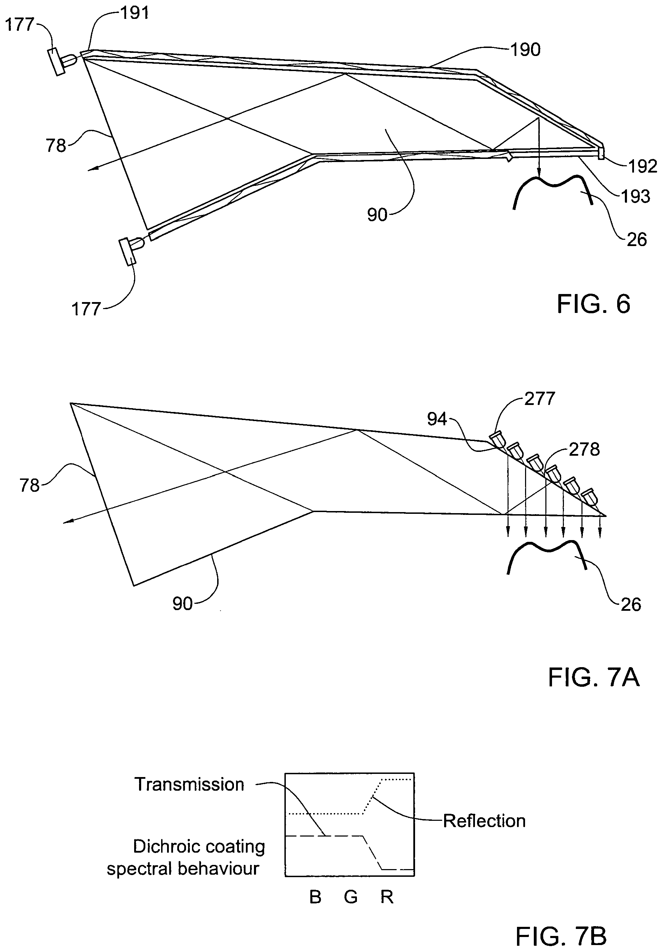

According to a second embodiment of the device **100,** the endoscope **46,** is also in the form of a probing member **90,** substantially as described with respect to the first embodiment, but with the difference that there are no LED's directly mounted thereon at the interface **78,** *mutatis mutandis.* In the second embodiment the delivery optics **73** is in the form of a disposable sleeve, shroud or sheath **190** that covers the outer surface the probing member **90,** as illustrated in Fig. 6. The sheath **190** is made from a waveguiding material, such as an acrylic polymer for example, capable of transmitting an illuminating radiation from the upstream face **191** of the sheath **190** therethrough and to the downstream face **192** thereto. The upstream face **191** is in the form of a peripheral surface around the interface **78.** The downstream face **192** is formed as a peripheral projection surrounding a window **193** comprised in said sheath **190.** The window **193** is in registry with the user interface end **79** of the probe **90.** A plurality of tri- colour LED's **177** for providing the coloured illumination to the object **26** are mounted on the device **100** just upstream of the sheath **190.** The tri-colour LED's **177** are each capable of providing an illumination radiation in one of three colours, typically red, green or blue, as controlled via the tri- colour sequence generator **74.** Alternatively, a plurality of LED's in three groups, each group providing one coloured illumination, may be provided. The LED's **177** are located outside of the main optics of the device **100,** and thus the LED's do not interfere with the other optical operations of the device **100** in particular including the transmission of the illuminating radiation for the confocal focusing operations, or in the transmission of reflected light from the object **26** to provide the 3D entity or the 2D colour entity. The LED's are mounted substantially opposite to the upstream face **191,** and thus, as illustrated in Fig. 6, light from each of the LED's **177** is transmitted by the waveguiding sheath **190** to downstream face **192** and thence to the object **26.** In this embodiment, the sheath **190** is particularly useful in maintaining hygienic conditions between one patient and the next, and avoids the need for sterilizing the probe **90,** since the sheath may be discarded after being used with one patient, and replaced with another sterilised sheath before conducting an intra-oral cavity survey with the next patient.

Preferably, the device **100** according to a variation of the second embodiment is further adapted for providing improved precision of the colour data obtained therewith, in a similar manner to that described herein for the fourth embodiment, *mutatis mutandis.*

In either one of the first or second embodiments, or variations thereof, a red laser may be used as the illumination source **28** for the main optics when creating the 3D entity. As such, this illumination means may also be used to obtain the red monochromatic image for the creation of the 2D colour image, by illuminating the object **26** and recording the image with the optical detector **60.** Accordingly, rather than tri- colour LED's or LED's or three different colours, it is only necessary to provide LED's adapted to provide only the remaining two colours, green and blue. A similar situation arises if the illumination source for the main optics **41** is a green or blue laser, wherein illuminating radiations in only the remaining two colours need to be provided, *mutatis mutandis.*

In these embodiments, the positioning of the illumination sources at the upstream end of the probe **90** where there is ample room rather than at the patient interface end **79** where space is tight.

According to a third embodiment of the device **100,** the endoscope **46** is also in the form of a probing member **90,** substantially as described with respect to the second embodiment with the following differences, *mutatis mutandis.* As illustrated in Fig. 7A, in the third embodiment the delivery optics **73** comprises a plurality of LED's **277** for providing the coloured illumination to the object **26.** In this embodiment, a red laser is used as the illumination source for the main optics when creating the 3D entity. As such, this illumination means is also used to obtain the red monochromatic image for the creation of the 2D colour image. Thus, the LED's **277** are each capable of providing an illumination radiation in either green or blue, as controlled via the tri- colour sequence generator **74.** The LED's **277** are located on the outer side of slanted face **94,** and thus the LED's do not interfere with the other optical operations of the device **100** in particular including the transmission of the illuminating radiation for the confocal focusing operations, or in the transmission of reflected light from the object **26** to provide the 3D entity or the 2D colour entity. The slanted face **94** comprises a dichroic coating **278** on the outer side thereof, which has relatively high reflectivity and low transmission properties with respect to red light, while having substantially high transmission characteristics for blue light and green light, as illustrated in Fig. 7B. Thus, as illustrated in Fig. 7A, light from each of the blue or green LED's **277** is transmitted, in turn, through the dichroic coating to interface **79** and thence to the object **26,** as controlled by the generator **74.** At the same time the dichroic coating permits internal reflection of the red radiation from the main optics **41** to the interface **79** and object **26,** and thus allows the 3D scan to be completed, as well as allowing the red monochromatic image of the object **26** to be taken by the device **100.** Optionally, rather than employing blue and green LED's, tri colour LED's may be used, and properly synchronized to illuminate with either green or blue light as controlled by generator **74.** Alternatively, the illumination source for the main optics **41** may be a green or blue laser, in which case the LED's are each capable of providing illumination in the remaining two colours, and in such cases the dichroic coating is adapted for allowing transmission of these remaining two colours while providing substantially high reflection for the illuminating laser of the main optics, in a similar manner to that described above for the red laser, *mutatis mutandis.*

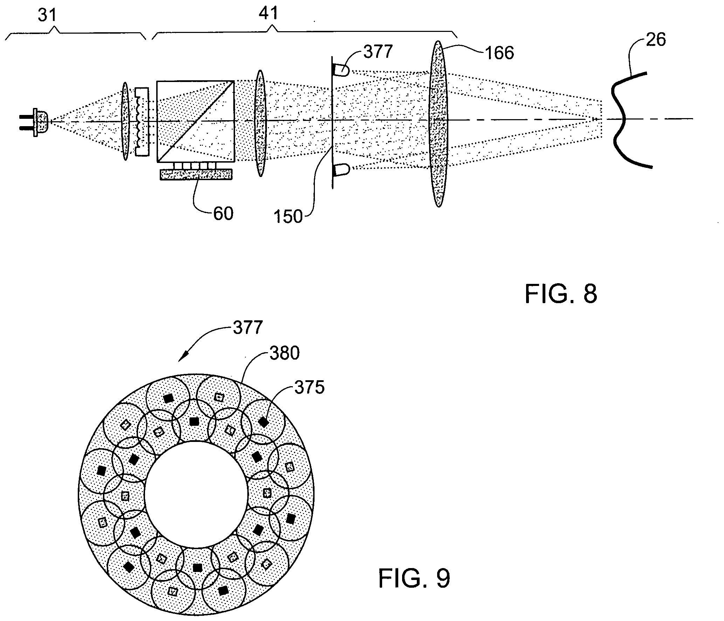

In a fourth embodiment of the device **100,** and referring to Fig. 8, tri-colour illumination is provided within the main focal optics **42,** in particular at the confocal system aperture stop, and facing the objective lens of the system. An advantage provided by this form of illumination is that the tri- colour illumination illuminates the object **26** through the downstream objective lens **142** in nearly collimated light, and thus the object illumination is highly uniform. The tri- colour light sources **377** may be mounted statically on the physical aperture stop at the aperture stop plane **150,** or alternatively they may be mounted on a retracting aperture stop, which also serves to stop down the system aperture in preview mode. In this embodiment, by placing the tri- colour light sources **377** at the aperture stop plane, wherein the light beam from the illumination source **31** narrows to a minimum within the main optics **41,** the external dimensions of the device **100** may still remain relatively compact.

Referring to Fig. 9, the tri- colour light sources **377** may comprise, for example, a plurality of tri- colour LED's **385** mounted onto a bracket **380.** The bracket **380** is typically annular, having a central aperture to allow illumination light from the illuminating unit **31** to pass therethrough and to the object **26,** and to allow light coming from the object **26** to pass therethrough and to the detection optics **60**, without being affected by the bracket **380**. At the same time, the bracket **380** positions the LED's in the required location upstream of objective lens **166.** The LED's are arranged in a spaced radial and circumferential manner as illustrated in Fig. 9 to provide the most uniform illumination of the object **26** possible with this arrangement. Typically, a red laser is used as the illumination source **31** for the main optics **41** when creating the 3D entity. As such, and as in other embodiments, this illumination means is also used to obtain the red monochromatic image for the creation of the 2D colour image. Thus, the LED's **385** are each capable of providing an illumination radiation in either green or blue, as controlled via the tri- colour sequence generator **74.** Alternatively, the illumination source for the main optics **41** may be a green or blue laser, in which case the LED's **385** are each capable of providing illumination in the remaining two colours, in a similar manner to that described above for the red laser, *mutatis mutandis.* Optionally, rather than employing blue and green LED's, tri colour LED's may be used, and properly synchronized to illuminate with either green or blue light as controlled by generator **74.** Further optionally, the LED's **385** may be used to provide, sequentially, all the required coloured illuminations, typically red, green and blue. Alternatively, the LED's **385** each provide illumination in one of at least three colours. Thus, some of the LED's **385** provide a blue illumination, while other LED's **385** provide green illumination, while yet other LED's **385** provide red illumination.

The device **100** according to a variation of the fourth embodiment, that is not part of the present invention, is further adapted for providing improved precision of the colour data obtained therewith. In this connection, the device **100** according to this variation of the fourth embodiment is adapted such that the tri- colour light sources **377** each illuminate the object **26** with as wide a depth of field as possible, i.e., at a low numerical aperture. Thus, each set of light sources **377** of the same colour, for example blue, illuminates a particular depth of the object **26** in the z-direction while substantially in focus. In contrast, the numerical aperture of the confocal system itself is relatively high to maximize accuracy of the depth measurements, and thus provides a relatively narrower depth of field.

Advantageously, the optical system downstream of the light sources **377,** in this embodiment the objective lens **166,** is chromatic, and in particular maximizes the chromatic dispersion therethrough. Alternatively or additionally, a chromatic dispersion element, for example an optically refractive block of suitable refractive index, may be provided along the optical path between the light sources **377** and the object **26.** Thus, each one of the different- coloured light sources **377** illuminates a different portion of the object **26** along the z-direction. The light sources **377** providing the blue illumination illuminate in focus a portion of the object **26** closest to the device **100,** and the light sources **377** providing the red illumination illuminate in focus a portion of the object **26** furthest from the device **100.** At the same time, the light sources **377** providing the green illumination illuminate in focus a portion of the object **26** intermediate the blue and red portions, and a non-illuminated gap may exists between the red and green, and between the green and blue illuminated portions, the depth of these gaps depending on the dispersion characteristics of the downstream optics. Advantageously, the light sources **377** are also adapted for providing illumination in colours intermediate in wavelengths such as to illuminate the aforesaid gaps in focus. Thus, the LED's **385** may be adapted for providing both such additional coloured illumination, or some of the LED's **385** may be adapted to provide coloured illumination at a first intermediate wavelength, while another set of LED's **385** may be adapted to provide coloured illumination at a second intermediate wavelength. For example, the first intermediate wavelength provides an illumination in aqua, and thus illuminates in focus at least a part of the gaps between the blue and green illuminated focused zones of the object **26,** while the second intermediate wavelength provides an illumination in amber, and thus illuminates in focus at least a part the gaps between the green and red illuminated focused zones. Of course, additional light sources may be used to provide further intermediate wavelengths and thus provide further depth cover illumination, in focus, of the object.

While the device **100** is used as a viewfinder, typically prior to taking a depth and colour scan of the object **26,** the above arrangement using at least five different coloured illuminations at a low numerical aperture, enables a much clearer and focused real-time colour image of the object **26** to be obtained. Thus when in operation in viewfinder mode (also known as "aiming mode", prior to the 3D scan event, while the dental practitioner is in the process of aiming the scanner onto the target dental surface, for example) the device **100** according to this variation of the fourth embodiment repeatedly illuminates the object **26** in cycles, wherein in each cycle the object **26** is separately illuminated in each of the five colours blue, aqua, green, amber, red, in quick succession, and each time a monochromatic image is obtained by the monochromatic image sensor in **60.** Each set of five monochromatic images is then analysed to provide a composite colour image, and this image is then displayed in substantially real time in the viewfinder display window in the control software, so that the succession of such composite images gives the appearance of a substantially real-time colour video feed of the object **26.**

Each of the monochrome images in any particular set corresponds to a particular illumination colour or wavelength, and thus the zone(s) of the object **26** within the depth of field corresponding to this illumination will be in focus, while the other parts of the object **26** will appear out of focus. Thus, each such image in the aforesaid set of images will contain a portion which has high precision focused image of a part of the object, for the particular illumination wavelength.

In forming a composite image for each set of images, the images are combined in such a way as to maximize the precision of the focused image and corresponding colour thereof. Thus, as an example that is not part of the present invention, suitable algorithms may be applied to each of the five images of a set to distinguish between the focused and unfocused the areas thereof. Such algorithms may employ, for example, techniques which apply FFT techniques to areas of the images, and which search for high frequency portions which correspond to focused areas. In any case, such algorithms, as well as software and hardware to accomplish the same are well known in the art. Then, the focused areas of each of the five images are merged to provide a monochrome composite substantially focused image of the object. Next, the images obtained using the red, green and blue illuminations are combined and converted to a corresponding luminescence/chroma (Y/C) image, and techniques for doing so are well known in the art. Finally, the luminescence component of the luminescence/chroma (Y/C) image is replaced with the aforesaid corresponding composite focus image, and the resulting new luminescence/chroma image is then transmitted to the display in the viewfinder.

For each set of images, prior to combining the corresponding red, green and blue images, these are preferably first scaled to compensate for magnification effects of the different wavelengths. Thus, the green image, and more so the blue image, needs to be scaled up to match the red image.

When the user is ready to take a depth and colour scan of the object **26,** having steered the device **100** into position with the aid of the viewfinder, the device **100** takes a depth scan in the z-direction as described herein, and either before or after the same, but in quick succession one with the other, takes a colour scan in a similar manner to that described above for the viewfinder mode, *mutatis mutandis.* Subsequently, the colour data and the depth data of the two scans can be combined to provide the full spatial and colour data for the surface scanned.

Advantageously, one or more colour scans may also be taken during the depth scan, and/or at the beginning and at the end of the depth scan. In one mode of operation, the depth scan is obtained by displacing the objective lens **166** along the z-direction in a continuous or stepped motion. Multiple colour scans can then be obtained by associating the colour sources **377** with the objective lens, so that these are also displaced along the z-direction. Accordingly, as the light sources **377** are moved in the z-direction towards the object **26** during the depth scan, at each different z-position in which a set of images is taken (concurrently with or alternately with the depth scan), each one of the coloured illuminations - red, green, blue and intermediate wavelengths - illuminates a progressively deeper part of the object along the z-direction. Of course, in some cases it is possible that at the downstream end of the depth scan the green and red illuminations completely overshoot the object **26,** and the corresponding images may be discarded or otherwise manipulated to provide a composite colour image at this station. Thus, a plurality of colour images can be obtained, each based on a different z-position, so that each illumination wavelength is used to illuminate in focus a different part (depth) of the object **26.** Advantageously, suitable algorithms that are not part of the present invention may be used to form a composite colour image of the set of colour images associated with a particular z-scan of the object **26** to provide even more precise and accurate colour image, than can then be combined with the depth data.

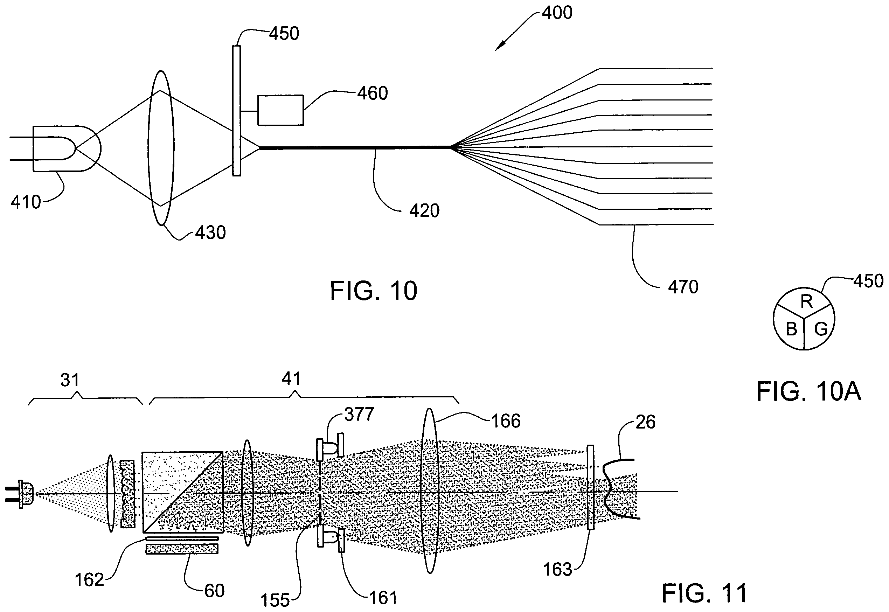

Alternatively, and referring to Fig. 10, the tri- colour light sources **377** may be replaced with a rotating filter illumination system **400.** The system **400** comprises a while light source **410,** such as for example white phosphorus InGaN LED's, and the light therefrom is focused onto an optical fiber bundle **420** by means of condenser optics **430**. Between the condenser optics **430** and the fiber bundle **420** is provided a rotating tri- colour filter **450**. As best seen in Fig. 10A, the filter **450** is divided into three coloured sections, comprising blue, green and red filters on adjacent sectors therein. The fiber bundle **420** is flared at the downstream end **470** to form the desired illumination pattern. Optionally, the downstream end **470** of the fibers may be mounted onto an annular bracket similar to bracket **380** illustrated in Fig. 9, at the aperture stop plane of the confocal optics. A suitable motor **460,** typically a stepper motor for example, drives the rotating filter such as to sequentially present each coloured filter to the light passing from the condenser optics **430** to the fiber bundle **420,** as synchronized with the sequence generator **74** (Fig. 1) to enable the detection optics **60** to capture images of the object 26 when selectively illuminated with each of the three colours. Optionally, if a red, blue or green illuminating radiation is used as the illumination source **31** for the main optics **41** when creating the 3D entity, then the rotating filter **450** only requires to comprise the remaining two colours, as discussed above for similar situations regarding the LED's, *mutatis mutandis.*

Preferably, the device **100** according to this variation of the fourth embodiment may be further adapted in a variation that is not part of the present invention for providing improved precision of the colour data obtained therewith, in a similar manner to that described herein for another variation of fourth embodiment, *mutatis mutandis.* In particular, the filter **450** is divided into five (or more if desired) coloured sections, comprising blue, aqua, green, amber and red filters on adjacent sectors therein.

A fifth embodiment of system **100** is substantially similar to the fourth embodiment as described herein, with the following difference, *mutatis mutandis.* In the fifth embodiment, and referring to Fig. 11, polarizers are provided at two locations in order to increase the image contrast. A first polarizing element **161** is located just downstream of the light sources **377** so as to polarize the light emitted from the light sources **377.** A second polarizing element **162** is located just upstream of the image sensor of the detection optics **60,** and is crossed with respect to the first polarizing element **161.** Further, a quarter waveplate **163** is provided just upstream of the object **26,** i.e. at the downstream end of the endoscope **46** (Fig. 4A). The first polarizing element **161** is typically annular, having a central aperture to allow illumination light from the illuminating unit **31** to pass therethrough and to the object, and to allow light coming from the object **26** to pass therethrough and to the detection optics **60,** without being affected by the polarizing element **161.** However, light that is reflected from the object **26** returns to the main confocal optics **42** in a crossed polarization state due to the effect of the quarter waveplate **163,** and thus reaches the detection optics **60** at substantially full intensity. However, any light reflected from the objective lens **166** of the confocal optics **42** is reflected at the same polarization state, and is therefore filtered out by the crossed polarizing element **162.** This arrangement serves as an effective signal to ghost enhancement system.

Preferably, the device **100** according to a variation of the fifth embodiment that is not part of the present invention is further adapted for providing improved precision of the colour data obtained therewith, in a similar manner to that described herein for the fourth embodiment, *mutatis mutandis.*

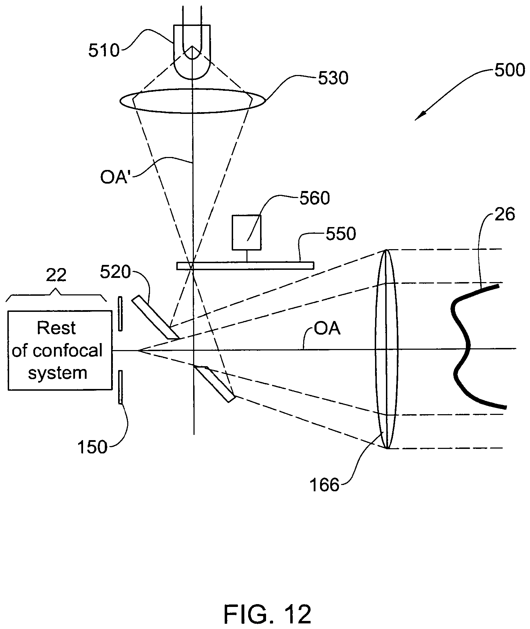

A sixth embodiment of the system **100** is substantially as described for the fourth embodiment, with the following difference, *mutatis mutandis.* In the sixth embodiment, and referring to Fig. 12, the tri- colour light sources **377** are replaced with a rotating filter illumination system **500.** The system **500** comprises a while light source **510,** such as for example white phosphorus InGaN LED's, and the light therefrom is focused onto a mirror **520** by means of condenser optics **530**. Between the condenser optics **530** and the mirror **520** is provided a rotating tri- colour filter **550,** which is similar to the filter **450** illustrated in Fig. 11, and thus comprises three coloured sections, comprising blue, green and red filters on adjacent sectors therein, and is actuated by motor **560**. The optical axis **OA** of the confocal optics **41** is orthogonal to the optical axis **OA'** of the light source **510** and condenser optics **530.** The mirror **520** is mounted between the aperture stop plane and the objective lens **166** of the confocal optics, and at an angle to the optical axis **OA** thereof and to the optical axis **OA'** of the light source **510** and condenser optics **530.** The mirror **520** is typically annular, having a central aperture aligned with optical axis **OA** to allow illumination light from the illuminating unit **31** to pass therethrough and to the object **26,** and to allow light coming from the object **26** to pass therethrough and to the detection optics **60,** without being affected by the mirror **520.** At the same time, the mirror **520** has sufficient reflecting surface to reflect light from the source **510** via objective lens **166** and to the object **26.** Optionally, if a red, blue or green illuminating radiation is used as the illumination source **31** for the main optics **41** when creating the 3D entity, then the rotating filter **550** only requires the remaining two colours, as discussed above for similar situations, *mutatis mutandis.*

Preferably, the device **100** according to a variation of the sixth embodiment that is not part of the invention is further adapted for providing improved precision of the colour data obtained therewith, in a similar manner to that described herein for the fourth embodiment, *mutatis mutandis.*

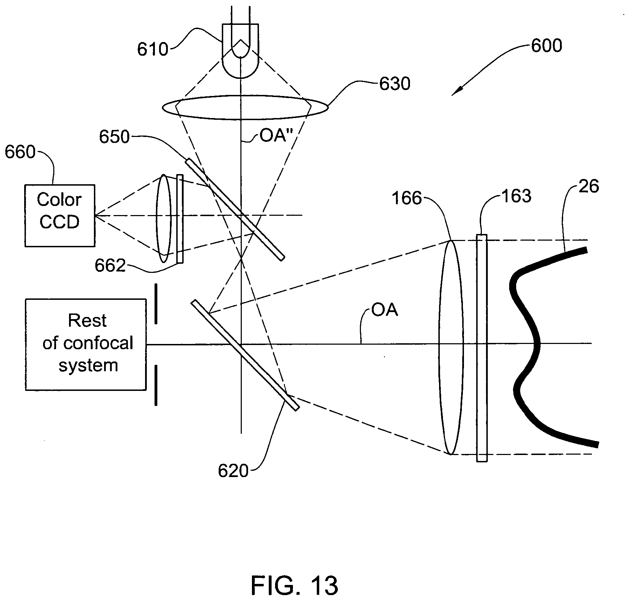

According to a second technique for providing the aforesaid 2D colour image, the object **26** is illuminated with a white light, and a colour CCD is used for receiving the light reflected from the object **26.** Thus, a seventh embodiment of the system **100** comprises a white light illumination system **600**, illustrated in Fig. 13. The system **600** comprises a while light source **610,** such as for example white phosphorus InGaN LED's, and the light therefrom is directed onto a flip mirror **620** via a polarizing beam splitter **650** by means of condenser optics **630.** The optical axis **OA** of the confocal optics **41** is orthogonal to the optical axis **OA"** of the light source **610** and condenser optics **630.** The mirror **620** is mounted between the aperture stop plane **155** and the objective lens **166** of the confocal optics, and at an angle to the optical axis **OA** thereof and to the optical axis **OA"** of the light source **610** and condenser optics **630.**

The mirror **620** is adapted to flip away from optical axis **OA** when the device **100** is being used for obtaining the 3D entity E. This allows illumination light from the illuminating unit **31** to pass therethrough and to the object **26,** and to allow light coming from the object **26** to pass therethrough and to the detection optics **60,** without being affected by the mirror **620**. When it is desired to take a 2D colour image, the mirror **620** is flipped down to the position shown in Fig. 13. Polarizing beam splitter **650** that polarizes white light from the source **610** and allows the same to pass therethrough and to mirror **620**, and thence to the object **26** via the confocal objective **166** and broadband quarter wave plate **163.** Light that is reflected from the object **26** returns to the mirror **620** in a crossed polarization state due to the effect of the quarter waveplate **163,** and thus reaches the colour CCD **660** (and associated detection optics - not shown) at substantially full intensity. However, any light reflected from the objective lens **166** of the confocal optics **42** is reflected at the same polarization state, and is therefore filtered out by a crossed polarizing element **662** just upstream of the CCD **660**. This arrangement serves as an effective signal to ghost enhancement system.

Alternatively, the CCD of the detection optics **60** is a colour CCD and is also used for the 2D scan. In such a case, flipping mirror **620** is replaced with a fixed mirror having a central aperture similar to mirror **520,** having a central aperture, as described for the sixth embodiment, mutatis mutandis.

In the seventh embodiment, the image capture device **80** and processing software **82** (Fig. 4b) automatically provide a 2D colour image comprising an array of data points having location **(X,Y)** and colour **(C)** information for each pixel of the image.

According to a third technique for providing the 2D colour image, the object is illuminated with a white light, and the light reflected from the object **26** is passed sequentially through one of three different coloured filters such as red, green ad blue. Each time a monochromatic image corresponding to each colour is captured via CCD **68** and the image capture device **80** (see Figs. 4A, 4B). Suitable processing software **82** combines the three images to provide a 2D colour image comprising an array of data points having location **(X,Y)** and colour **(C)** information for each pixel of the image.

According to a fourth technique for providing the colour image, the main illumination source **31** of device **100** comprises suitable means for providing the three different coloured illuminations. In one embodiment, the illumination source **31** comprises three different lasers, each one providing an illumination radiation at a different desired colour, red green or blue. In another embodiment, a suitable white light illumination means is provided, coupled to a suitable rotating tri- colour filter, similar to the filters described above, *mutatis mutandis.* In each case, suitable control means are provided, adapted to illuminate the object **26** with each coloured radiation in turn, and the 2D coloured image is obtained in a similar fashion to that described above, *mutatis mutandis.* The object is also illuminated with one of the coloured illuminations in order to provide the 3D surface topology data.

In each of the embodiments described herein, the illumination radiation that is used for obtaining the 2D colour image is injected into the optical axis OA of the confocal optics **42** without affecting the operation thereof or degrading the 3D image capture.

The endoscope **46,** the illumination unit **31,** the main optics **41,** colour illumination **71** and tri- colour sequence genetrator are preferably included together in a unitary device, typically a hand-held device. The device preferably includes also the detector optics **60,** though the latter may be connected to the remainder of the device via a suitable optical link such as a fibre optics cable.

For all embodiments, the data representative of the surface topology and colour, i.e., entity **I,** may be transmitted through an appropriate data port, e.g. a modem **88** (Fig. 4B), through any communication network, e.g. telephone line **90,** to a recipient (not shown) e.g. to an off-site CAD/CAM apparatus (not shown).

By capturing, in this manner, an image from two or more angular locations around the structure, inside the intraoral cavity of a patient, e.g. in the case of a teeth segment from the buccal direction, from the lingual direction and optionally from above the teeth, an accurate colour three-dimensional representation of the teeth segment may be reconstructed. This may allow a virtual reconstruction of the three-dimensional structure in a computerized environment or a physical reconstruction in a CAD/CAM apparatus.

While the present invention has been described in the context of a particular embodiment of an optical scanner that uses confocal focusing techniques for obtaining the 3D entity, the device may comprise in examples that are not part of the invention any other confocal focusing arrangement, for example as described in WO 00/08415. In fact, any suitable means for providing 3D scanning can be used in examples that are not part of the invention, so long as the 3D scan and the colour 2D scan correspond substantially to the same object or portion thereof being scanned, and the same frames of references are maintained. Typically the scans are executed in relatively quick succession, and by the same or different image capturing means such as CCD's that are arranged such that the colour 2D image substantially corresponds to the 3D entity. This enables colour values at particular x, y coordinates of the 2D colour image to be matched to the same x, y coordinates of the 3D image which also have a z coordinate.

The embodiments illustrated herein are particularly useful for determining the three-dimensional structure of a teeth segment, particularly a teeth segment where at least one tooth or portion of tooth is missing for the purpose of generating data of such a segment for subsequent use in design or manufacture of a prosthesis of the missing at least one tooth or portion, e.g. a crown, or a bridge, or a dental restoration or a filing.

In the method claims that follow, alphabetic characters and Roman numerals used to designate claim steps are provided for convenience only and do not imply any particular order of performing the steps.

Finally, it should be noted that the word "comprising" as used throughout the appended claims is to be interpreted to mean "including but not limited to".

## Claims

1. A handheld intraoral scanning device (100) for determining the surface topology and associated colour of at least a portion of a three dimensional structure (26) in the intraoral cavity of a patient, the handheld intraoral scanning device comprising:
scanning means for performing a three-dimensional scan and adapted for providing depth data of said portion, said depth data being in a Cartesian X, Y, Z frame with depth Z values for an array range of X, Y points corresponding to a two-dimensional reference array substantially orthogonal to a depth direction, wherein said operation of said scanning means is based on confocal imaging techniques, and wherein said scanning means comprises:
a probing member (90) with a sensing face (97);
first illumination means (31) for providing a first array of incident light beams (48) transmitted towards the structure (26), the light beams (48) propagating along an optical path through said probing member (90) and, arranged in an X, Y plane, in the depth Z direction, along the Z axis, towards the structure (26) to generate illuminated spots (52) on said portion at different Xᵢ, Yᵢ locations along said depth direction, wherein said first array is defined within said reference array;
a light focusing optics (42) defining one or more focal planes forward said sensing face at a position changeable by said optics (42), each light beam (48) having its focus on one of said one or more focal planes;
a translation mechanism (72) for displacing said focal plane relative to the structure (26) along said Z axis defined by the propagation of the incident light beams (48);
a first detector (68) having an array of sensing elements for measuring intensity of each of a plurality of light beams (54) returning from said spots (52) propagating through the optical path in a direction opposite to that of the incident light beams (48);
a processor (24) coupled to said detector (68) for determining for each light beam that generates a said spot (52), for each said Xᵢ, Yᵢ location, a spot-specific position Zᵢ, being a position of a respective focal plane of said one or more focal planes yielding maximum measured intensity of the returned light beam (54), and based on the determined spot- specific positions, generating data representative of the topology of said portion as a three-dimensional numerical entity (E) comprising an array of X, Y, Z points; and
colour illumination means (74);
**characterised in that** the device (100) further comprises:
imaging means adapted for providing, for an array of X, Y points, two-dimensional colour image data of said portion associated with said reference array using the colour illumination means (74);
wherein the device (100) is adapted for maintaining a spatial disposition with respect to said portion that is substantially fixed during operation of said scanning means and said imaging means; and
wherein the processor (24) is adapted for outputting the two-dimensional colour image data so that a viewfinder to assist a user in aiming the handheld intraoral scanning device for the three-dimensional scan is provided;
wherein the processor (24) is adapted for conformally mapping colour values at each X, Y coordinate of the two-dimensional colour image data provided by the imaging means to spatial coordinates in the depth data having the same X, Y coordinates to provide a numerical entity (I) representing the colour and surface topology of the portion, the numerical entity (I) comprising a plurality of data points, each data point comprising three-dimensional surface coordinate data and colour data mapped therewith;
wherein the processor (24) is further adapted for outputting a rendering of a full colour three-dimensional image of the portion based on the numerical (I) entity representing the colour and surface topology of the portion.

2. The handheld intraoral scanning device (100) according to claim 1, wherein said device (100) is adapted for providing a time interval between acquisition of said depth data and acquisition of said colour image data such that substantially no significant relative movement between said device and said portion occurs.

3. The handheld intraoral scanning device (100) according to claim 2, wherein said time interval is between about 0 seconds to about 100 milliseconds, preferably between 0 and 50 milliseconds, and more preferably between 0 and 20 milliseconds.

4. The handheld intraoral scanning device (100) according to claim 1, further comprising processing means for associating said colour data with said depth data for corresponding data points of said reference array.

5. The handheld intraoral scanning device (100) according to claim 1, wherein said first array is arranged to provide depth data at a plurality of predetermined spatial coordinates substantially corresponding to the spatial disposition of said incident light beams (48).

6. The handheld intraoral scanning device (100) according to claim 1, wherein the imaging means are arranged to provide colour data at a plurality of spatial coordinates substantially corresponding to the spatial coordinates of said first array.

7. The handheld intraoral scanning device (100) according to claim 6, comprising colour illumination means adapted for providing three second illuminating radiations, each of a different colour.

8. The handheld intraoral scanning device (100) according to claim 7, wherein said colour illumination means comprises any one of the following:
- second illumination means for providing said three second illuminating radiations, each of a different colour;
- second illumination means for providing two said second illuminating radiations, and wherein said first illumination means provides another said second illuminating radiation each said second illuminating radiation being of a different colour.

9. The handheld intraoral scanning device (100) according to claim 8, wherein said second illumination means comprise radiation transmission elements that are configured to be located out of the path of said light beams or said returned light beam at least within said light focusing optics.

10. The handheld intraoral scanning device (100) according to claim 9, wherein said probing member (90) is made from a light transmissive material having an upstream optical interface with said light focusing optics (42) and a reflective face for reflecting light between said optical interface and said sensing face (97).

11. The handheld intraoral scanning device (100) according to claim 10, wherein said second illumination means are optically coupled to said optical interface for selectively transmitting illuminating radiations in at least two colours to said portion via said sensing face (97).

12. The handheld intraoral scanning device (100) according to claim 10, wherein said colour illumination means comprises second illumination means for providing two said second illuminating radiations, and wherein said first illumination means provides another said second illuminating radiation each said second illuminating radiation being of a different colour.

13. The handheld intraoral scanning device (100) according to claim 12, wherein said probing member (90) comprises a removable sheath (190) having an inner surface substantially complementary to an outer surface of said probing member (90), and having a window (93) in registry with said sensing face (97), wherein said sheath (190) is made from a waveguiding material and is adapted to transmit said light from said second illuminating means from an upstream face (91) thereof to a downstream face (92) associated with said window (93).

14. The handheld intraoral scanning device (100) according to claim 13, wherein said second illumination means are optically coupled to said upstream face (91) for selectively transmitting said second illuminating radiations in at least two colours to said portion via said downstream face (92).

15. The handheld intraoral scanning device (100) according to claim 10, wherein said reflective face comprises a dichroic coating (278), having relatively high reflectivity and low optical transmission properties for a said second illuminating radiation provided by said first illumination means, and relatively low reflectivity and high optical transmission properties for the two said second illuminating radiations provided by said second illumination means.

16. The handheld intraoral scanning device (100) according to claim 9, wherein said second illumination means are adapted for providing second illuminating radiations within said light focusing optics (42).

17. The handheld intraoral scanning device (100) according to claim 16, wherein said second illumination means are adapted for providing second illuminating radiations at an aperture stop plane (150) of said light focusing optics (42).

18. The handheld intraoral scanning device (100) according to claim 16, further comprising:
- a first polarizing element (161) located just downstream of said illumination means so as to polarize the light emitted therefrom;
- a second polarizing element (162) located just upstream of said first detector, wherein said second polarizing element (162) is crossed with respect to the first polarizing element (161); and
- a quarter waveplate (163) at the downstream end of said device.

19. The handheld intraoral scanning device (100) according to claim 16, wherein said illumination means are adapted for selective movement in the depth direction.

20. The handheld intraoral scanning device (100) according to claim 16, comprising a mirror inclined to the optical axis of said light focusing optics (42), said mirror having an aperture configured to allow said light beams and said returning light beams to pass therethrough without being optically affected by said mirror, and wherein said second illumination means comprises at least one white illumination source optically coupled with suitable colour filters, said filters selectively providing illumination radiation in each colour in cooperation with said white illumination source, wherein said mirror is coupled to said white illumination source to direct radiation therefrom along said optical axis.

21. The handheld intraoral scanning device (100) according to claim 9, wherein said second illumination means are in the form of any one of the following:
- suitable LED's, comprising at least one LED for providing illumination radiation in each colour;
- suitable LED's, comprising at least one white illumination source optically coupled with suitable colour filters, said filters selectively providing illumination radiation in each colour in cooperation with said white illumination source.

22. The handheld intraoral scanning device (100) according to claim 7, wherein said first detector is adapted for selectively measuring intensity of each second illuminating radiation after reflection from said portion.

23. The handheld intraoral scanning device (100) according to claim 5, wherein said imaging means is configured to operate based on any one of the following:
― illuminating said portion with substantially white illumination radiation, and capturing a colour image of said portion, wherein said white illuminating radiation is provided in the form of a second array of incident light beams (48) transmitted towards the portion along an optical path through said probing unit to generate illuminated spots on said portion along said depth direction, wherein said second array is defined within a reference frame;
― illuminating said portion with substantially white illumination radiation, selectively passing radiation reflected from said portion through a number of colour filters, capturing a monochromatic image of said portion corresponding to each said filter, and combining the monochromatic images to create a full colour image, wherein said illuminating radiation is provided in the form of a second array of incident light beams transmitted towards the portion along an optical path through said probing unit to generate illuminated spots on said portion along said depth direction, wherein said second array is defined within said reference frame;
― illuminating said portion with three differently-coloured illumination radiations, capturing a monochromatic image of said portion corresponding to each said illuminating radiation, and combining the monochromatic images to create a full colour image, wherein each said illuminating radiation is provided in the form of a second array of incident light beams transmitted towards the portion along an optical path through said probing unit to generate illuminated spots on said portion along said depth direction, wherein said second array is defined within said reference frame, and wherein said illuminating radiations are provided by said first illumination source.

24. The handheld intraoral scanning device (100) according to claim 23, wherein said second array is arranged to provide colour data at a plurality of spatial coordinates substantially corresponding to the spatial coordinates of said first array.

25. The handheld intraoral scanning device (100) according to claim 24, wherein said imaging means comprises:-
― white illumination radiation means;
― a second detector (66) having an array of sensing elements for measuring intensity of said white illuminating radiation after reflection from said portion.

26. The handheld intraoral scanning device (100) according to claim 7, further comprising a tri-colour sequence generator (74) for controlling the illumination of said portion with said second illuminating radiations.

27. The handheld intraoral scanning device (100) according to claim 26, further comprising at least one of
― a tri-colour sequence generator (74) for controlling the illumination of said portion with said second illuminating radiations;
― a unit for generating manufacturing data based on said numerical entity for transmission to CAD/CAM device based on said entity
― a communication port of a communication medium.

28. The handheld intraoral scanning device (100) according to claim 27, adapted for determining colour and surface topology of a teeth portion.

29. Method for determining the surface topology and associated colour of at least a portion of a three dimensional structure in the intraoral cavity, comprising:
(a) providing, in a three-dimensional scan, depth data of said portion, said depth data being in a Cartesian X, Y, Z frame depth Z values for an array range of X, Y points and corresponding to a two-dimensional reference array substantially orthogonal to a depth direction;
wherein step (a) comprises:
(i) providing a first array of incident light beams (48) defined within said reference array propagating in an optical path leading through a focusing optics (42) and through a sensing face (97); and, arranged in an X, Y plane, propagating in the depth Z direction along the Z axis towards the structure; the focusing optics (42) defining one or more focal planes forward said sensing face in a position changeable by said optics, each light beam having its focus on one of said one or more focal plane; the beams generating a plurality of illuminated spots (52) on the structure at different Xᵢ, Yᵢ locations;
(ii) detecting intensity of returned light beams (54) propagating from each of these spots along the optical path in a direction opposite to that of the incident light;
(iii) repeating steps (i) and (ii) a plurality of times, each time changing position of the focal plane relative to the structure along the Z axis;
(iv) for each of the illuminated spots, for each said Xᵢ, Yᵢ location, determining a spot-specific position Zᵢ, being the position of the respective focal plane yielding a maximum measured intensity of a respective returned light beam; and
(v) generating, based on the determined spot-specific positions data representative of the topology of said portion as a three-dimensional numerical entity (E) comprising an array of X, Y, Z points;
**characterized in that** the method comprises the steps of
(b) providing two-dimensional colour image data of said portion associated with said reference array using colour illumination means (74);
(c) ensuring that a spatial disposition with respect to said portion during steps (a) and (b) is substantially fixed; and
(d) conformally mapping said colour data to said depth data for said reference array comprising mapping colour values at each X, Y coordinate of the two-dimensional colour image data to spatial coordinates in the depth data having the same X, Y coordinates to provide a numerical entity (I) representing the colour and surface topology of the portion, the further numerical entity (I) comprising a plurality of data points, each data point comprising three-dimensional surface coordinate data and colour data mapped therewith,
wherein the method further comprises:
outputting a rendering of a full colour three-dimensional image of the portion based on the numerical entity representing the colour and surface topology of the portion.

30. Method according to claim 29, wherein in step (c), a minimum time interval is allowed between acquisition of said depth data and acquisition of said image data.

31. Method according to claim 30, wherein said time interval is between about 0 seconds to about 100 milliseconds, preferably between 0 and 50 milliseconds, and more preferably between 0 and 20 milliseconds.

32. Method according to claim 29, wherein said depth data is provided using confocal imaging techniques.

33. Method according to claim 29, wherein step (b) is based on any one of the following:
(A) illuminating said portion with three differently-coloured illumination radiations, said illumination radiations being combinable to produce white radiation, capturing a monochromatic image of said portion corresponding to each said illuminating radiation, and combining the monochromatic images to create a full colour image, wherein each said illuminating radiation is provided in the form of a second array of incident light beams (48) transmitted towards the portion along an optical path through said probing unit to generate illuminated spots on said portion along said depth direction, wherein said second array is defined within a reference frame;
(B) illuminating said portion with substantially white illumination radiation, selectively passing radiation reflected from said portion through a number of colour filters, capturing a monochromatic image of said portion corresponding to each said filter, and combining the monochromatic images to create a full colour image, wherein said illuminating radiation is provided in the form of a second array of incident light beams transmitted towards the portion along an optical path through said probing unit to generate illuminated spots on said portion along said depth direction, wherein said second array is defined within said reference frame;
(C) illuminating said portion with three differently-coloured illumination radiations, capturing a monochromatic image of said portion corresponding to each said illuminating radiation, and combining the monochromatic images to create a full colour image, wherein each said illuminating radiation is provided in the form of a second array of incident light beams (48) transmitted towards the portion along an optical path through said probing unit to generate illuminated spots on said portion along said depth direction, wherein said second array is defined within said reference frame, and wherein said illuminating radiations are provided by said first illumination source.

34. Method according to claim 29, wherein said second array is arranged to provide colour data at a plurality of spatial coordinates substantially corresponding to the spatial coordinates of said first array.

35. Method according to claim 29, further comprising at least one of the following steps:
(A1) using the virtual three-dimensional and colour model corresponding to said portion for constructing an object to be fitted within said structure;
(B1) converting the virtual three-dimensional and colour model corresponding to said portion into a form transmissible through a communication medium to recipient.

36. Method according to claim 35, wherein in step (A1) said structure is a teeth segment with at least one missing tooth or a portion of a tooth and said object is said at least one missing tooth or the portion of the tooth.

37. Method according to claim 29, comprising repeating steps (i) to (v) for two different surfaces of said structure to provide surface topologies thereof; and combining the surface topologies to obtain colour and topological data representative of said structure.

38. Method according to claim 36, for reconstruction of topology of a teeth portion, comprising:
determining surface topologies of at least a buccal surface and a lingual surface of the teeth portion;
combining the surface topologies to obtain data representative of a three-dimensional structure of said teeth portion.

39. Method according to claim 29, wherein the method further comprises:
outputting the two-dimensional colour image data so that a viewfinder to assist a user in aiming the handheld intraoral scanning device for the three-dimensional scan is provided.

## Patentansprüche

1. Handgehaltenes intraorales Scan-Gerät (100) zum Bestimmen der Oberflächentopologie und assoziierten Farbe von mindestens einem Teil einer dreidimensionalen Struktur (26) in der Mundhöhle eines Patienten, wobei das handgehaltene intraorale Scan-Gerät Folgendes umfasst:
Scan-Mittel zum Durchführen eines dreidimensionalen Scans und ausgelegt zum Liefern von Tiefendaten des genannten Teils, wobei die genannten Tiefendaten in einem kartesischen X-, Y-, Z-Rahmen mit Tiefen-Z-Werten für einen Array-Bereich von X-, Y-Punkten vorliegen, die einem zweidimensionalen Referenz-Array entsprechen, das im Wesentlichen orthogonal zu einer Tiefenrichtung ist, wobei der genannte Betrieb des genannten Scan-Mittels auf konfokalen Bildgebungstechniken basiert und wobei das genannte Scan-Mittel Folgendes umfasst:
ein Sondierungselement (90) mit einer Abtastfläche (97);
erste Beleuchtungsmittel (31) zum Bereitstellen eines ersten Array von einfallenden Lichtstrahlen (48), die in Richtung der Struktur (26) übertragen werden, wobei sich die Lichtstrahlen (48) entlang eines optischen Pfades durch das genannte Sondierungselement (90) ausbreiten und in einer X-, Y-Ebene in der Tiefen-Z-Richtung entlang der Z-Achse in Richtung der Struktur (26) angeordnet sind, um beleuchtete Flecken (52) auf dem genannten Teil an verschiedenen Xi-, Yi-Orten entlang der genannten Tiefenrichtung zu erzeugen, wobei das genannte erste Array innerhalb des genannten Referenzarray definiert ist;
eine Lichtfokussierungsoptik (42), die eine oder mehrere Fokalebenen vor der genannten Abtastfläche an einer durch die genannte Optik (42) veränderbaren Position definiert, wobei jeder Lichtstrahl (48) seinen Fokus auf einer der genannten ein oder mehreren Fokalebenen hat;
einen Translationsmechanismus (72) zum Verschieben der genannten Fokalebene relativ zu der Struktur (26) entlang der genannten, durch die Ausbreitung der einfallenden Lichtstrahlen (48) definierten Z-Achse;
einen ersten Detektor (68) mit einem Array von Abtastelementen zum Messen der Intensität von jedem von mehreren Lichtstrahlen (54), die von den genannten Flecken (52) zurückkehren und sich durch den optischen Pfad in einer Richtung entgegengesetzt zu der der einfallenden Lichtstrahlen (48) ausbreiten;
einen Prozessor (24), der mit dem genannten Detektor (68) gekoppelt ist, zum Bestimmen für jeden Lichtstrahl, der einen genannten Fleck (52) erzeugt, für jeden genannten Xi-, Yi-Ort, einer fleckspezifischen Position Zi, die eine Position einer jeweiligen Fokalebene der genannten ein oder mehreren Fokalebenen ist, die eine maximale gemessene Intensität des zurückgeführten Lichtstrahls (54) ergibt, und zum Erzeugen, auf der Basis der bestimmten fleckspezifischen Positionen, von Daten, die für die Topologie des genannten Teils repräsentativ sind, als eine dreidimensionale numerische Entität (E), die ein Array von X-, Y- und Z-Punkten umfasst; und
Farbbeleuchtungsmittel (74);
**dadurch gekennzeichnet, dass** das Gerät (100) ferner Folgendes umfasst:
Bildgebungsmittel, ausgelegt zum Liefern, für ein Array von X-, Y-Punkten, von zweidimensionalen Farbbilddaten des genannten, mit dem genannten Referenzarray assoziierten Teils mit dem Farbbeleuchtungsmittel (74);
wobei das Gerät (100) zum Beibehalten einer räumlichen Anordnung in Bezug auf den genanten Teil ausgelegt ist, die während des Betriebs des genannten Scan-Mittels und des genannten Bildgebungsmittels im Wesentlichen fest ist; und
wobei der Prozessor (24) zum Ausgeben der zweidimensionalen Farbbilddaten ausgelegt ist, so dass ein Sucher zum Unterstützen eines Benutzers beim Zielen des tragbaren intraoralen Scan-Geräts für den dreidimensionalen Scan geliefert wird;
wobei der Prozessor (24) zum konformen Abbilden von Farbwerten an jeder X-, Y-Koordinate der von dem Bildgebungsmittel gelieferten zweidimensionalen Farbbilddaten auf Raumkoordinaten in den Tiefendaten mit denselben X-, Y-Koordinaten ausgelegt ist, um eine numerische Entität (I) zu liefern, die die Farbe und Oberflächentopologie des Teils darstellt, wobei die numerische Entität (I) mehrere Datenpunkte umfasst, wobei jeder Datenpunkt dreidimensionale Oberflächenkoordinatendaten und damit abgebildete Farbdaten umfasst;
wobei der Prozessor (24) ferner zum Ausgeben eines Renderings eines dreidimensionalen Vollfarbbildes des Teils auf der Basis der numerischen Entität (I) ausgelegt ist, die die Farbe und Oberflächentopologie des Teils darstellt.

2. Handgehaltenes intraorales Scan-Gerät (100) nach Anspruch 1, wobei das genannte Gerät (100) zum Liefern eines Zeitintervalls zwischen der Erfassung der genannten Tiefendaten und der Erfassung der genannten Farbbilddaten ausgelegt ist, so dass im Wesentlichen keine signifikante Relativbewegung zwischen dem genannten Gerät und dem genannten Teil auftritt.

3. Handgehaltenes intraorales Scan-Gerät (100) nach Anspruch 2, wobei das genannte Zeitintervall zwischen etwa 0 Sekunden und etwa 100 Millisekunden, vorzugsweise zwischen 0 und 50 Millisekunden und bevorzugter zwischen 0 und 20 Millisekunden liegt.

4. Handgehaltenes intraorales Scan-Gerät (100) nach Anspruch 1, das ferner Verarbeitungsmittel zum Assoziieren der genannten Farbdaten mit den genannten Tiefendaten für entsprechende Datenpunkte des genannten Referenzarray umfasst.

5. Handgehaltenes intraorales Scan-Gerät (100) nach Anspruch 1, wobei das genannte erste Array zum Liefern von Tiefendaten an mehreren vorbestimmten Raumkoordinaten ausgelegt ist, die im Wesentlichen der räumlichen Anordnung der genannten einfallenden Lichtstrahlen (48) entsprechen.

6. Handgehaltenes intraorales Scan-Gerät (100) nach Anspruch 1, wobei die Bildgebungsmittel zum Liefern von Farbdaten an mehreren Raumkoordinaten ausgelegt sind, die im Wesentlichen den Raumkoordinaten des genannten ersten Array entsprechen.

7. Handgehaltenes intraorales Scan-Gerät (100) nach Anspruch 6, das Farbbeleuchtungsmittel umfasst, die zum Liefern von drei zweiten Beleuchtungsstrahlungen ausgelegt sind, die jeweils eine andere Farbe haben.

8. Handgehaltenes intraorales Scan-Gerät (100) nach Anspruch 7, wobei das genannte Farbbeleuchtungsmittel eines der Folgenden umfasst:
- zweite Beleuchtungsmittel zum Liefern der genannten drei zweiten Beleuchtungsstrahlungen, die jeweils eine andere Farbe haben;
- zweite Beleuchtungsmittel zum Liefern von zwei genannten zweiten Beleuchtungsstrahlungen, und wobei das genannte erste Beleuchtungsmittel eine andere genannte zweite Beleuchtungsstrahlung liefert, wobei jede genannte zweite Beleuchtungsstrahlung eine andere Farbe hat.

9. Handgehaltenes intraorales Scan-Gerät (100) nach Anspruch 8, wobei die genannten zweiten Beleuchtungsmittel Strahlungstransmissionselemente umfassen, die so konfiguriert sind, dass sie sich zumindest innerhalb der genannten Lichtfokussierungsoptik außerhalb des Pfades der genannten Lichtstrahlen oder des genannten zurückgeführten Lichtstrahls befinden.

10. Handgehaltenes intraorales Scan-Gerät (100) nach Anspruch 9, wobei das genannte Sondierungselement (90) aus einem lichtdurchlässigen Material mit einer stromaufwärts gelegenen optischen Schnittstelle mit der genannten Lichtfokussierungsoptik (42) und einer reflektierenden Fläche zum Reflektieren von Licht zwischen der genannten optischen Schnittstelle und der genannten Abtastfläche (97) gefertigt ist.

11. Handgehaltenes intraorales Scan-Gerät (100) nach Anspruch 10, wobei die genannten zweiten Beleuchtungsmittel optisch mit der genannten optischen Schnittstelle gekoppelt sind, um selektiv Beleuchtungsstrahlungen in mindestens zwei Farben über die genannte Abtastfläche (97) zu dem genannten Teil zu übertragen.

12. Handgehaltenes intraorales Scan-Gerät (100) nach Anspruch 10, wobei das genannte Farbbeleuchtungsmittel zweite Beleuchtungsmittel zum Liefern von zwei genannten zweiten Beleuchtungsstrahlungen umfasst, und wobei das genannte erste Beleuchtungsmittel eine andere genannte zweite Beleuchtungsstrahlung liefert, wobei jede genannte zweite Beleuchtungsstrahlung eine andere Farbe hat.

13. Handgehaltenes intraorales Scan-Gerät (100) nach Anspruch 12, wobei das genannte Sondierungselement (90) eine abnehmbare Hülle (190) mit einer Innenfläche im Wesentlichen komplementär zu einer Außenfläche des genannten Sondierungselements (90) und einem mit der genannten Abtastfläche (97) ausgerichteten Fenster (93) umfasst, wobei die genannte Hülle (190) aus einem wellenleitenden Material gefertigt und zum Übertragen des genannten Lichts von dem genannten zweiten Beleuchtungsmittel von einer stromaufwärts gelegenen Fläche (91) davon zu einer mit dem genannten Fenster (93) assoziierten stromabwärts gelegenen Fläche (92) ausgelegt ist.

14. Handgehaltenes intraorales Scan-Gerät (100) nach Anspruch 13, wobei die genannten zweiten Beleuchtungsmittel optisch mit der genannten stromaufwärts gelegenen Fläche (91) zum selektiven Übertragen der genannten zweiten Beleuchtungsstrahlungen in mindestens zwei Farben über die genannte stromabwärts gelegene Fläche (92) zu dem genannten Teil gekoppelt sind.

15. Handgehaltenes intraorales Scan-Gerät (100) nach Anspruch 10, wobei die genannte reflektierende Fläche eine dichroitische Beschichtung (278) mit einem relativ hohen Reflexionsvermögen und niedrigen optischen Transmissionseigenschaften für eine von dem genannten ersten Beleuchtungsmittel gelieferte genannte zweite Beleuchtungsstrahlung und einem relativ niedrigen Reflexionsvermögen und hohen optischen Transmissionseigenschaften für die von den genannten zweiten Beleuchtungsmitteln gelieferten beiden genannten zweiten Beleuchtungsstrahlungen aufweist.

16. Handgehaltenes intraorales Scan-Gerät (100) nach Anspruch 9, wobei die genannten zweiten Beleuchtungsmittel zum Liefern zweiter Beleuchtungsstrahlungen innerhalb der Lichtfokussierungsoptik (42) ausgelegt sind.

17. Handgehaltenes intraorales Scan-Gerät (100) nach Anspruch 16, wobei die genannten zweiten Beleuchtungsmittel zum Liefern zweiter Beleuchtungsstrahlungen an einer Aperturblendenebene (150) der genannten Lichtfokussierungsoptik (42) ausgelegt sind.

18. Handgehaltenes intraorales Scan-Gerät (100) nach Anspruch 16, das ferner Folgendes umfasst:
- ein erstes Polarisationselement (161), das sich unmittelbar stromabwärts der genannten Beleuchtungsmittel befindet, um das davon emittierte Licht zu polarisieren;
- ein zweites Polarisationselement (162), das sich unmittelbar stromaufwärts des genannten ersten Detektors befindet, wobei das genannte zweite Polarisationselement (162) in Bezug auf das erste Polarisationselement (161) gekreuzt ist; und
- eine Viertelwellenplatte (163) am stromabwärts gelegenen Ende des genannten Geräts.

19. Handgehaltenes intraorales Scan-Gerät (100) nach Anspruch 16, wobei die genannten Beleuchtungsmittel für eine selektive Bewegung in der Tiefenrichtung ausgelegt sind.

20. Handgehaltenes intraorales Scan-Gerät (100) nach Anspruch 16, umfassend einen Spiegel, der zur optischen Achse der genannten Lichtfokussierungsoptik (42) geneigt ist, wobei der genannte Spiegel eine Apertur aufweist, die so konfiguriert ist, dass die genannten Lichtstrahlen und die genannten zurückkehrenden Lichtstrahlen durch sie passieren können, ohne durch den genannten Spiegel optisch beeinflusst zu werden, und wobei das genannte zweite Beleuchtungsmittel mindestens eine weiße Beleuchtungsquelle umfasst, die optisch mit geeigneten Farbfiltern gekoppelt ist, wobei die genannten Filter selektiv Beleuchtungsstrahlung in jeder Farbe in Zusammenwirkung mit der genannten weißen Beleuchtungsquelle liefern, wobei der genannte Spiegel mit der genannten weißen Beleuchtungsquelle gekoppelt ist, um Strahlung davon entlang der genannten optischen Achse zu lenken.

21. Handgehaltenes intraorales Scan-Gerät (100) nach Anspruch 9, wobei die genannten zweiten Beleuchtungsmittel in Form von einem der Folgenden vorliegen:
- geeigneten LEDs, die mindestens eine LED zum Liefern von Beleuchtungsstrahlung in jeder Farbe umfassen;
- geeigneten LEDs, die mindestens eine weiße Beleuchtungsquelle umfassen, die optisch mit geeigneten Farbfiltern gekoppelt ist, wobei die genannten Filter selektiv Beleuchtungsstrahlung in jeder Farbe in Zusammenwirkung mit der genannten weißen Beleuchtungsquelle liefern.

22. Handgehaltenes intraorales Scan-Gerät (100) nach Anspruch 7, wobei der genannte erste Detektor zum selektiven Messen der Intensität jeder zweiten Beleuchtungsstrahlung nach der Reflexion von dem genannten Teil ausgelegt ist.

23. Handgehaltenes intraorales Scan-Gerät (100) nach Anspruch 5, wobei das genannte Bildgebungsmittel zum Arbeiten auf der Basis von einem der Folgenden konfiguriert ist:
- Beleuchten des genannten Teils mit im Wesentlichen weißer Beleuchtungsstrahlung und Aufnehmen eines Farbbildes des genannten Teils, wobei die genannte weiße Beleuchtungsstrahlung in Form eines zweiten Array von einfallenden Lichtstrahlen (48) geliefert wird, die entlang eines optischen Pfades durch die genannte Sondierungseinheit in Richtung des Teils übertragen werden, um beleuchtete Flecken auf dem genannten Teil entlang der genannten Tiefenrichtung zu erzeugen, wobei das genannte zweite Array innerhalb eines Referenzrahmens definiert ist;
- Beleuchten des genannten Teils mit im Wesentlichen weißer Beleuchtungsstrahlung, selektives Durchlassen von von dem genannten Teil reflektierter Strahlung durch eine Anzahl von Farbfiltern, Aufnehmen eines monochromatischen Bildes des genannten Teils, das jedem genannten Filter entspricht, und Kombinieren der monochromatischen Bilder, um ein Vollfarbbild zu erzeugen, wobei die genannte Beleuchtungsstrahlung in Form eines zweiten Array von einfallenden Lichtstrahlen geliefert wird, die in Richtung des Teils entlang eines optischen Pfads durch die genannte Sondierungseinheit übertragen werden, um beleuchtete Flecken auf dem genannten Teil entlang der genannten Tiefenrichtung zu erzeugen, wobei das genannte zweite Array innerhalb des genannten Bezugsrahmens definiert ist;
- Beleuchten des genannten Teils mit drei verschiedenfarbigen Beleuchtungsstrahlungen, Aufnehmen eines monochromatischen Bildes des genannten Teils, das jeder genannten Beleuchtungsstrahlung entspricht, und Kombinieren der monochromatischen Bilder, um ein Vollfarbbild zu erzeugen, wobei jede genannte Beleuchtungsstrahlung in Form eines zweiten Array von einfallenden Lichtstrahlen geliefert wird, die entlang eines optischen Pfades durch die genannte Sondierungseinheit in Richtung des Teils übertragen werden, um beleuchtete Flecken auf dem genannten Teil entlang der genannten Tiefenrichtung zu erzeugen, wobei das genannte zweite Array innerhalb des genannten Referenzrahmens definiert ist und wobei die genannten Beleuchtungsstrahlungen durch die genannte erste Beleuchtungsquelle geliefert werden.

24. Handgehaltenes intraorales Scan-Gerät (100) nach Anspruch 23, wobei das genannte zweite Array zum Liefern von Farbdaten an mehreren Raumkoordinaten ausgelegt ist, die im Wesentlichen den Raumkoordinaten des genannten ersten Array entsprechen.

25. Handgehaltenes intraorales Scan-Gerät (100) nach Anspruch 24, wobei das genannte Bildgebungsmittel Folgendes umfasst:
- Strahlungsmittel für weiße Beleuchtung;
- einen zweiten Detektor (66) mit einem Array von Abtastelementen zum Messen der Intensität der genannten weißen Beleuchtungsstrahlung nach der Reflexion von dem genannten Teil.

26. Handgehaltenes intraorales Scan-Gerät (100) nach Anspruch 7, das ferner einen Dreifarbsequenzgenerator (74) zum Steuern der Beleuchtung des genannten Teils mit den genannten zweiten Beleuchtungsstrahlungen umfasst.

27. Handgehaltenes intraorales Scan-Gerät (100) nach Anspruch 26, das ferner mindestens eines der Folgenden umfasst:
- einen Dreifarbsequenzgenerator (74) zum Steuern der Beleuchtung des genannten Teils mit den genannten zweiten Beleuchtungsstrahlungen;
- eine Einheit zum Erzeugen von Herstellungsdaten auf der Basis der genannten numerischen Entität zur Übertragung zu einem CAD/CAM-Gerät auf der Basis der genannten Entität;
- einen Kommunikationsport eines
Kommunikationsmediums.

28. Handgehaltenes intraorales Scan-Gerät (100) nach Anspruch 27, ausgelegt zum Bestimmen von Farbe und Oberflächentopologie eines Zahnteils.

29. Verfahren zum Bestimmen der Oberflächentopologie und assoziierten Farbe von mindestens einem Teil einer dreidimensionalen Struktur in der Mundhöhle, das Folgendes beinhaltet:
(a) Liefern, in einem dreidimensionalen Scan, von Tiefendaten des genannten Teils, wobei die genannten Tiefendaten in einem kartesischen X-, Y-, Z-Rahmen mit Tiefen-Z-Werten für einen Array-Bereich von X-, Y-Punkten vorliegen und einem zweidimensionalen Referenzarray im Wesentlichen orthogonal zu einer Tiefenrichtung entsprechen;
wobei Schritt (a) Folgendes beinhaltet:
(i) Liefern eines ersten Array von einfallenden Lichtstrahlen (48), die innerhalb des genannten Referenzarray definiert sind und sich in einem optischen Pfad ausbreiten, der durch eine Fokussierungsoptik (42) und durch eine Abtastfläche (97) führt; und die sich, in einer X-, Y-Ebene angeordnet, in der Tiefen-Z-Richtung entlang der Z-Achse in Richtung der Struktur ausbreiten;
wobei die Fokussierungsoptik (42) eine oder mehrere Fokalebenen vor der genannten Abtastfläche in einer durch die genannte Optik veränderbaren Position definiert, wobei jeder Lichtstrahl seinen Fokus auf einer der genannten ein oder mehreren Fokalebenen hat; wobei die Strahlen mehrere beleuchtete Flecken (52) auf der Struktur an unterschiedlichen Xi-, Yi-Orten erzeugen;
(ii) Erkennen der Intensität von zurückgeführten Lichtstrahlen (54), die sich von jedem dieser Flecken entlang des optischen Pfads in einer Richtung entgegengesetzt zu der des einfallenden Lichts ausbreiten;
(iii) mehrmaliges Wiederholen der Schritte (i) und (ii), wobei sich jedes Mal die Position der Fokalebene relativ zu der Struktur entlang der Z-Achse ändert;
(iv) Bestimmen, für jeden der beleuchteten Flecken, für jeden genannten Xᵢ-, Yᵢ-Ort, einer fleckenspezifischen Position Zi, die die Position der jeweiligen Fokalebene ist, die eine maximale gemessene Intensität eines jeweiligen zurückgeführten Lichtstrahls ergibt; und
(v) Erzeugen, auf der Basis der bestimmten fleckspezifischen Positionen, von Daten, die für die Topologie des genannten Teils repräsentativ sind, als eine dreidimensionale numerische Entität (E), die ein Array von X-, Y- und Z-Punkten umfasst;
**dadurch gekennzeichnet, dass** das Verfahren die folgenden Schritte beinhaltet:
(b) Liefern von zweidimensionalen Farbbilddaten des genannten, mit dem genannten Referenzarray assoziierten Teil mittels Farbbeleuchtungsmitteln (74);
(c) Sicherstellen, dass eine räumliche Anordnung in Bezug auf den genannten Teil während der Schritte (a) und (b) im Wesentlichen fest ist; und
(d) konformes Abbilden der genannten Farbdaten auf die genannten Tiefendaten für das genannte Referenzarray, das das Abbilden von Farbwerten an jeder X-, Y-Koordinate der zweidimensionalen Farbbilddaten auf Raumkoordinaten in den Tiefendaten mit denselben X-, Y-Koordinaten beinhaltet, um eine numerische Entität (I) zu liefern, die die Farbe und Oberflächentopologie des Teils darstellt, wobei die weitere numerische Entität (I) mehrere Datenpunkte umfasst, wobei jeder Datenpunkt dreidimensionale Oberflächenkoordinatendaten und damit abgebildete Farbdaten umfasst,
wobei das Verfahren ferner Folgendes beinhaltet:
Ausgeben eines Renderings eines dreidimensionalen Vollfarbbildes des Teils auf der Basis der numerischen Entität, die die Farbe und Oberflächentopologie des Teils repräsentiert.

30. Verfahren nach Anspruch 29, wobei in Schritt (c) ein Mindestzeitintervall zwischen der Erfassung der genannten Tiefendaten und der Erfassung der genannten Bilddaten zugelassen wird.

31. Verfahren nach Anspruch 30, wobei das genannte Zeitintervall zwischen etwa 0 Sekunden und etwa 100 Millisekunden, vorzugsweise zwischen 0 und 50 Millisekunden und bevorzugter zwischen 0 und 20 Millisekunden liegt.

32. Verfahren nach Anspruch 29, wobei die genannten Tiefendaten mittels konfokaler Bildgebungsverfahren geliefert werden.

33. Verfahren nach Anspruch 29, wobei Schritt (b) auf einem der Folgenden basiert:
(A) Beleuchten des genannten Teils mit drei verschiedenfarbigen Beleuchtungsstrahlen, wobei die genannten Beleuchtungsstrahlungen kombiniert werden können, um weiße Strahlung zu erzeugen, Aufnehmen eines monochromatischen Bildes des genannten Teils, das jeder genannten Beleuchtungsstrahlung entspricht, und Kombinieren der monochromatischen Bilder, um ein Vollfarbbild zu erzeugen, wobei jede genannte Beleuchtungsstrahlung in Form eines zweiten Array von einfallenden Lichtstrahlen (48) geliefert wird, die entlang eines optischen Pfades durch die genannte Sondierungseinheit in Richtung des genannten Teils übertragen werden, um beleuchtete Flecken auf dem genannten Teil entlang der genannten Tiefenrichtung zu erzeugen, wobei das genannte zweite Array innerhalb eines Referenzrahmens definiert ist;
(B) Beleuchten des genannten Teils mit im Wesentlichen weißer Beleuchtungsstrahlung, selektives Durchlassen von von dem genannten Teil reflektierter Strahlung durch eine Anzahl von Farbfiltern, Aufnehmen eines monochromatischen Bildes des genannten Teils, das jedem genannten Filter entspricht, und Kombinieren der monochromatischen Bilder, um ein Vollfarbbild zu erzeugen, wobei die genannte Beleuchtungsstrahlung in Form eines zweiten Array von einfallenden Lichtstrahlen geliefert wird, die in Richtung des Teils entlang eines optischen Pfades durch die genannte Sondierungseinheit übertragen werden, um beleuchtete Flecken auf dem genannten Teil entlang der genannten Tiefenrichtung zu erzeugen, wobei das genannte zweite Array innerhalb des genannten Referenzrahmens definiert ist;
(C) Beleuchten des genannten Teils mit drei verschiedenfarbigen Beleuchtungsstrahlungen, Aufnehmen eines monochromatischen Bildes des genannten Teils, das jeder genannten Beleuchtungsstrahlung entspricht, und Kombinieren der monochromatischen Bilder, um ein Vollfarbbild zu erzeugen, wobei jede genannte Beleuchtungsstrahlung in Form eines zweiten Array von einfallenden Lichtstrahlen (48) geliefert wird, die entlang eines optischen Pfads durch die genannte Sondierungseinheit in Richtung des Teils übertragen werden, um beleuchtete Flecken auf dem genannten Teil entlang der genannten Tiefenrichtung zu erzeugen, wobei das genannte zweite Array innerhalb des genannten Referenzrahmens definiert ist und wobei die genannten Beleuchtungsstrahlungen von der genannten ersten Beleuchtungsquelle geliefert werden.

34. Verfahren nach Anspruch 29, wobei das genannte zweite Array zum Liefern von Farbdaten an mehreren Raumkoordinaten ausgelegt ist, die im Wesentlichen den Raumkoordinaten des genannten ersten Array entsprechen.

35. Verfahren nach Anspruch 29, das ferner mindestens einen der folgenden Schritte beinhaltet:
(A1) Verwenden des virtuellen dreidimensionalen und Farbmodells, das dem genannten Teil entspricht, zum Konstruieren eines Objekts, das in die genannte Struktur eingepasst werden soll;
(B1) Umwandeln des virtuellen dreidimensionalen und Farbmodells, das dem genannten Teil entspricht, in eine Form, die über ein Kommunikationsmedium an einen Empfänger übertragen werden kann.

36. Verfahren nach Anspruch 35, wobei in Schritt (A1) die genannte Struktur ein Zahnsegment mit mindestens einem fehlenden Zahn oder einem Teil eines Zahns ist und das genannte Objekt der genannte mindestens eine fehlende Zahn oder der Teil des Zahns ist.

37. Verfahren nach Anspruch 29, das das Wiederholen der Schritte (i) bis (v) für zwei verschiedene Oberflächen der genannten Struktur, um deren Oberflächentopologien zu liefern; und das Kombinieren der Oberflächentopologien beinhaltet, um für die genannte Struktur repräsentative Farb- und Topologiedaten zu erhalten.

38. Verfahren nach Anspruch 36 zum Rekonstruieren der Topologie eines Zahnteils, das Folgendes beinhaltet:
Bestimmen von Oberflächentopologien von mindestens einer bukkalen Oberfläche und einer lingualen Oberfläche des Zahnteils;
Kombinieren der Oberflächentopologien, um Daten zu erhalten, die für eine dreidimensionale Struktur des genannten Zahnteils repräsentativ sind.

39. Verfahren nach Anspruch 29, wobei das Verfahren ferner Folgendes beinhaltet:
Ausgeben der zweidimensionalen Farbbilddaten, so dass ein Sucher zum Unterstützen eines Benutzers beim Zielen des handgehaltenen intraoralen Scan-Geräts für den dreidimensionalen Scan geliefert wird.

## Revendications

1. Dispositif de balayage intra-oral portatif (100) destiné à déterminer la topologie de surface et la couleur associée d'au moins une partie d'une structure tridimensionnelle (26) dans la cavité intra-orale d'un patient, le dispositif de balayage intra-oral portatif comprenant :
un moyen de balayage destiné à réaliser un balayage tridimensionnel et conçu pour fournir des données de profondeur de ladite partie, lesdites données de profondeur étant dans un cadre cartésien X, Y, Z avec des valeurs de profondeur Z pour une plage de réseau de points X, Y correspondant à un réseau de référence bidimensionnel substantiellement perpendiculaire à une direction de profondeur, dans lequel ledit fonctionnement dudit moyen de balayage est basé sur des techniques d'imagerie confocale, et dans lequel ledit moyen de balayage comprend :
un élément de sondage (90) avec une face de détection (97) ;
un premier moyen d'éclairage (31) destiné à fournir un premier réseau de faisceaux de lumière incidents (48) émis vers la structure (26), les faisceaux de lumière (48) se propageant le long d'un chemin optique à travers ledit élément de sondage (90) et, agencé dans un plan X, Y, dans la direction de profondeur Z, le long de l'axe Z, vers la structure (26) pour générer des taches éclairées (52) sur ladite partie au niveau de différents emplacements Xi, Yi le long de ladite direction de longueur, dans lequel ledit premier réseau est défini dans les limites dudit réseau de référence ;
un élément optique de focalisation de lumière (42) définissant un ou plusieurs plans focaux en avant de ladite face de détection au niveau d'une position modifiable par ledit élément optique (42), chaque faisceau de lumière (48) ayant son foyer sur l'un desdits un ou plusieurs plans focaux ;
un mécanisme de translation (72) destiné à déplacer ledit plan focal par rapport à la structure (26) le long dudit axe Z défini par la propagation des faisceaux de lumière incidents (48) ;
un premier détecteur (68) ayant un réseau d'éléments de détection destiné à mesurer l'intensité de chacun d'une pluralité de faisceaux de lumière (54) réfléchis à partir desdites taches (52) se propageant par le chemin optique dans une direction opposée à celle des faisceaux de lumière incidents (48) ;
un processeur (24) couplé audit détecteur (68) destiné à déterminer pour chaque faisceau de lumière qui génère une desdites taches (52), pour chacun desdits emplacements Xi, Yi, une position spécifique d'une tache Zi, étant une position d'un plan focal respectif desdits un ou plusieurs plans focaux produisant une intensité mesurée maximale du faisceau de lumière réfléchis (54), et sur la base des positions spécifiques d'une tache déterminées, générer des données représentatives de la topologie de ladite partie comme une entité numérique tridimensionnelle (E) comprenant un réseau de points X, Y, Z ; et
un moyen d'éclairage en couleurs (74) ;
**caractérisé en ce que** le dispositif (100) comprend en outre :
un moyen d'imagerie conçu pour fournir, pour un réseau de points X, Y, des données d'image en couleurs bidimensionnelle de ladite partie associée audit réseau de référence en utilisant le moyen d'éclairage en couleurs (74) ;
dans lequel le dispositif (100) est conçu pour maintenir une disposition spatiale par rapport à ladite partie qui est substantiellement fixe pendant le fonctionnement dudit moyen de balayage et dudit moyen d'imagerie ; et
dans lequel le processeur (24) est conçu pour sortir les données d'image en couleurs bidimensionnelle de sorte qu'un viseur pour aider un utilisateur à pointer le dispositif de balayage intra-oral portatif pour le balayage tridimensionnel est fourni ;
dans lequel le processeur (24) est conçu pour faire correspondre de manière conforme des valeurs de couleur au niveau de chaque coordonnée X, Y des données d'image en couleurs bidimensionnelle fournies par le moyen d'imagerie avec des coordonnées spatiales dans les données de profondeur ayant les mêmes coordonnées X, Y pour fournir une entité numérique (I) représentant la couleur et la topologie de surface de la partie, l'entité numérique (I) comprenant une pluralité de points de données, chaque point de donnée comprenant des données de coordonnée de surface tridimensionnelle et des données de couleur mises en correspondance avec celles-ci ;
dans lequel le processeur (24) est conçu en outre pour sortir un rendu d'une image tridimensionnelle en couleurs de la partie sur la base de l'entité numérique (I) représentant la couleur et la topologie de surface de la partie.

2. Dispositif de balayage intra-oral portatif (100) selon la revendication 1, dans lequel ledit dispositif (100) est conçu pour fournir un intervalle de temps entre l'acquisition desdites données de profondeur et l'acquisition desdites données d'image en couleurs de sorte que substantiellement aucun mouvement relatif significatif entre ledit dispositif et ladite partie n'a lieu.

3. Dispositif de balayage intra-oral portatif (100) selon la revendication 2, dans lequel ledit intervalle de temps est entre environ 0 secondes à environ 100 millisecondes, de manière préférée entre 0 et 50 millisecondes, et de manière plus préférée entre 0 et 20 millisecondes.

4. Dispositif de balayage intra-oral portatif (100) selon la revendication 1, comprenant en outre un moyen de traitement destiné à associer lesdites données de couleur avec lesdites données de profondeur pour les points de données correspondants dudit réseau de référence.

5. Dispositif de balayage intra-oral portatif (100) selon la revendication 1, dans lequel ledit premier réseau est agencé pour fournir des données de profondeur au niveau d'une pluralité de coordonnées spatiales prédéterminées correspondant substantiellement à la disposition spatiale desdits faisceaux de lumière incidents (48).

6. Dispositif de balayage intra-oral portatif (100) selon la revendication 1, dans lequel les moyens d'imagerie sont agencés pour fournir des données de couleur au niveau d'une pluralité de coordonnées spatiales correspondant substantiellement aux coordonnées spatiales dudit premier réseau.

7. Dispositif de balayage intra-oral portatif (100) selon la revendication 6, comprenant un moyen d'éclairage en couleurs conçu pour fournir trois deuxièmes rayonnements d'éclairage, chacun d'une couleur différente.

8. Dispositif de balayage intra-oral portatif (100) selon la revendication 7, dans lequel ledit moyen d'éclairage de couleur comprend un moyen quelconque parmi les suivants :
- des deuxièmes moyens d'éclairage destinés à fournir lesdits trois deuxièmes rayonnements d'éclairage, chacun d'une couleur différente ;
- des deuxièmes moyens d'éclairage destinés à fournir deux desdits deuxièmes rayonnements d'éclairage, et dans lequel ledit premier moyen d'éclairage fournit un autre desdits deuxièmes rayonnements d'éclairage, chacun desdits deuxièmes rayonnements d'éclairage étant d'une couleur différente.

9. Dispositif de balayage intra-oral portatif (100) selon la revendication 8, dans lequel lesdits deuxièmes moyens d'éclairage comprennent des éléments d'émission de rayonnement qui sont configurés pour être situés hors du chemin desdits faisceaux de lumière ou dudit faisceau de lumière réfléchi au moins dans les limites dudit élément optique de focalisation de lumière.

10. Dispositif de balayage intra-oral portatif (100) selon la revendication 9, dans lequel ledit élément de sondage (90) est fabriqué à partir d'un matériau émetteur de lumière ayant une interface optique en amont avec ledit élément optique de focalisation de lumière (42) et une face réfléchissante destinée à réfléchir la lumière entre ladite interface optique et ladite face de détection (97).

11. Dispositif de balayage intra-oral portatif (100) selon la revendication 10, dans lequel lesdits deuxièmes moyens d'éclairage sont couplés de manière optique à ladite interface optique pour émettre de manière sélective des rayonnements d'éclairage dans au moins deux couleurs à ladite partie par le biais de ladite face de détection (97) .

12. Dispositif de balayage intra-oral portatif (100) selon la revendication 10, dans lequel ledit moyen d'éclairage de couleur comprend des deuxièmes moyens d'éclairage destinés à fournir deux desdits deuxièmes rayonnements d'éclairage, et dans lequel ledit premier moyen d'éclairage fournit un autre desdits deuxièmes rayonnements d'éclairage, chacun desdits deuxièmes rayonnements d'éclairage étant d'une couleur différente.

13. Dispositif de balayage intra-oral portatif (100) selon la revendication 12, dans lequel ledit élément de sondage (90) comprend une gaine amovible (190) ayant une surface interne substantiellement complémentaire d'une surface externe dudit élément de sondage (90), et ayant une fenêtre (93) en alignement avec ladite face de détection (97), dans lequel ladite gaine (190) est fabriquée à partir d'un matériau de guidage d'ondes et est conçue pour émettre ladite lumière dudit deuxième moyen d'éclairage d'une face en amont (91) de celui-ci à une face en aval (92) associée à ladite fenêtre (93).

14. Dispositif de balayage intra-oral portatif (100) selon la revendication 13, dans lequel lesdits deuxièmes moyens d'éclairage sont couplés de manière optique à ladite face en amont (91) pour émettre de manière sélective lesdits deuxièmes rayonnements d'éclairage dans au moins deux couleurs à ladite partie par le biais de ladite face en aval (92).

15. Dispositif de balayage intra-oral portatif (100) selon la revendication 10, dans lequel ladite face réfléchissante comprend un revêtement dichroïque (278), ayant un pouvoir réfléchissant relativement élevé et des propriétés d'émission optique faibles pour un desdits deuxièmes rayonnements d'éclairage fournis par ledit premier moyen d'éclairage, et un pouvoir réfléchissant relativement faible et des propriétés d'émission optique élevées pour les deux desdits deuxièmes rayonnements d'éclairage fournis par lesdits deuxièmes moyens d'éclairage.

16. Dispositif de balayage intra-oral portatif (100) selon la revendication 9, dans lequel lesdits deuxièmes moyens d'éclairage sont conçus pour fournir des deuxièmes rayonnements d'éclairage dans les limites dudit élément optique de focalisation de lumière (42).

17. Dispositif de balayage intra-oral portatif (100) selon la revendication 16, dans lequel lesdits deuxièmes moyens d'éclairage sont conçus pour fournir des deuxièmes rayonnements d'éclairage au niveau d'un plan de diaphragme (150) dudit élément de focalisation de lumière (42).

18. Dispositif de balayage intra-oral portatif (100) selon la revendication 16, comprenant en outre :
- un premier élément de polarisation (161) situé juste en aval dudit moyen d'éclairage de manière à polariser la lumière émise à partir de celui-ci ;
- un deuxième élément de polarisation (162) situé juste en amont dudit premier détecteur, dans lequel ledit deuxième élément de polarisation (162) est croisé par rapport au premier élément de polarisation (161); et
- une lame de quart d'onde (163) au niveau de l'extrémité aval dudit dispositif.

19. Dispositif de balayage intra-oral portatif (100) selon la revendication 16, dans lequel lesdits moyens d'éclairage sont conçus pour un mouvement sélectif dans la direction de profondeur.

20. Dispositif de balayage intra-oral portatif (100) selon la revendication 16, comprenant un miroir incliné par rapport à l'axe optique dudit élément de focalisation de lumière (42), ledit miroir ayant une ouverture configurée pour permettre que lesdits faisceaux de lumière et lesdits faisceaux de lumière réfléchis traversent sans être affectés de manière optique par ledit miroir, et dans lequel ledit deuxième moyen d'éclairage comprend au moins une source d'éclairage blanc couplée de manière optique avec des filtres colorés adaptés, lesdits filtres fournissant de manière sélective un rayonnement d'éclairage dans chaque couleur en coopération avec ladite source d'éclairage blanc, dans lequel ledit miroir est couplé à ladite source d'éclairage blanc pour diriger le rayonnement à partir de celle-ci le long dudit axe optique.

21. Dispositif de balayage intra-oral portatif (100) selon la revendication 9, dans lequel lesdits deuxièmes moyens d'éclairage prennent l'une quelconque des formes suivantes :
- des DEL adaptées, comprenant au moins une DEL pour fournir un rayonnement d'éclairage dans chaque couleur ;
- des DEL adaptées, comprenant au moins une source d'éclairage blanc couplée de manière optique avec des filtres colorés adaptés, lesdits filtres fournissant de manière sélective un rayonnement d'éclairage dans chaque couleur en coopération avec ladite source d'éclairage blanc.

22. Dispositif de balayage intra-oral portatif (100) selon la revendication 7, dans lequel ledit premier détecteur est conçu pour mesurer de manière sélective l'intensité de chaque deuxième rayonnement d'éclairage après sa réflexion à partir de ladite partie.

23. Dispositif de balayage intra-oral portatif (100) selon la revendication 5, dans lequel ledit moyen d'imagerie est configuré pour fonctionner sur la base de l'un quelconque des éclairages suivants :
- l'éclairage de ladite partie avec un rayonnement d'éclairage substantiellement blanc, et la capture d'une image en couleurs de ladite partie, dans lequel ledit rayonnement d'éclairage blanc est fourni sous la forme d'un deuxième réseau de faisceaux de lumière incidents (48) émis vers la partie le long d'un chemin optique à travers ladite unité de sondage pour générer des taches éclairées sur ladite partie le long de ladite direction de profondeur, dans lequel ledit deuxième réseau est défini dans les limites d'un cadre de référence ;
- l'éclairage de ladite partie avec un rayonnement d'éclairage substantiellement blanc, le passage sélectif du rayonnement réfléchi à partir de ladite partie à travers un certain nombre de filtres colorés, la capture d'une image monochrome de ladite partie correspondant à chacun desdits filtres, et la combinaison des images monochromes pour créer une image en pleines couleurs, dans lequel chaque rayonnement d'éclairage est fourni sous la forme d'un deuxième réseau de faisceaux de lumière incidents émis vers la partie le long d'un chemin optique à travers ladite unité de sondage pour générer des taches éclairées sur ladite partie le long de ladite direction de profondeur, dans lequel ledit deuxième réseau est défini dans les limites dudit cadre de référence ;
- l'éclairage de ladite partie avec trois rayonnements d'éclairage de couleurs différentes, la capture d'une image monochrome de ladite partie correspondant à chacun desdits rayonnements d'éclairage, et la combinaison des images monochromes pour créer une image en pleines couleurs, dans lequel chacun desdits rayonnements d'éclairage est fourni sous la forme d'un deuxième réseau de faisceaux de lumière incidents émis vers la partie le long d'un chemin optique à travers ladite unité de sondage pour générer des taches éclairées sur ladite partie le long de ladite direction de profondeur, dans lequel ledit deuxième réseau est défini dans les limites dudit cadre de référence, et dans lequel lesdits rayonnements d'éclairage sont fournis par ladite première source d'éclairage.

24. Dispositif de balayage intra-oral portatif (100) selon la revendication 23, dans lequel ledit deuxième réseau est agencé pour fournir des données de couleur au niveau d'une pluralité de coordonnées spatiales correspondant substantiellement aux coordonnées spatiales dudit premier réseau.

25. Dispositif de balayage intra-oral portatif (100) selon la revendication 24, dans lequel ledit moyen d'imagerie comprend :-
- un moyen de rayonnement d'éclairage blanc ;
- un deuxième détecteur (66) ayant un réseau d'éléments de détection destinés à mesurer l'intensité dudit rayonnement d'éclairage blanc après sa réflexion à partir de ladite partie.

26. Dispositif de balayage intra-oral portatif (100) selon la revendication 7, comprenant en outre un générateur de séquence tricolore (74) destiné à commander l'éclairage de ladite partie avec lesdits deuxièmes rayonnements d'éclairage.

27. Dispositif de balayage intra-oral portatif (100) selon la revendication 26, comprenant en outre au moins l'un des éléments suivants :
- un générateur de séquence tricolore (74) destiné à commander l'éclairage de ladite partie avec lesdits deuxièmes rayonnements d'éclairage ;
- une unité destinée à générer des données d'usinage sur la base de ladite entité numérique pour l'émission à un dispositif CAD/CAM sur la base de ladite entité
- un port de communication d'un support de communication.

28. Dispositif de balayage intra-oral portatif (100) selon la revendication 27, conçu pour déterminer la couleur et la topologie de surface d'une partie de dents.

29. Procédé destiné à déterminer la topologie de surface et la couleur associée d'au moins une partie d'une structure tridimensionnelle dans la cavité intra-orale, comprenant :
(a) la fourniture, dans un balayage tridimensionnel, de données de profondeur de ladite partie, lesdites données de profondeur étant des valeurs de profondeur Z dans un cadre cartésien X, Y, Z pour une plage de réseau de points X, Y et correspondant à un réseau de référence bidimensionnel substantiellement perpendiculaire à une direction de profondeur ;
dans lequel l'étape (a) comprend :
(i) la fourniture d'un premier réseau de faisceaux de lumière incidents (48) définis dans les limites du réseau de référence se propageant sur un chemin optique traversant un élément optique de focalisation (42) et traversant une face de détection (97) ; et, agencé dans un plan X, Y, se propageant dans la direction de profondeur Z le long de l'axe Z vers la structure ;
l'élément optique de focalisation (42) définissant un ou plusieurs plans focaux en avant de ladite face de détection dans une position modifiable par ledit élément optique, chaque faisceau de lumière ayant son foyer sur l'un desdits un ou plusieurs plans focaux ; les faisceaux générant une pluralité de taches éclairées (52) sur la structure au niveau de différents emplacements Xi, Yi ;
(ii) la détection de l'intensité des faisceaux de lumière réfléchis (54) se propageant à partir de chacune de ces taches le long du chemin optique dans une direction opposée à celle de la lumière incidente ;
(iii) la répétition des étapes (i) et (ii) une pluralité de fois, chaque fois modifiant la position du plan focal par rapport à la structure le long de l'axe Z ;
(iv) pour chacune des taches éclairées, pour chacun desdits emplacements Xi, Yi, la détermination d'une position spécifique d'une tache Zi, étant la position du plan focal respectif produisant une intensité mesurée maximale d'un faisceau de lumière réfléchi respectif ;
et
(v) la génération, sur la base des positions spécifiques d'une tache déterminées, de données représentatives de la topologie de ladite partie comme une entité numérique tridimensionnelle (E) comprenant un réseau de points X, Y, Z ;
**caractérisé en ce que** le procédé comprend les étapes suivantes :
(b) la fourniture de données d'image en couleurs bidimensionnelle de ladite partie associée audit réseau de référence en utilisant un moyen d'éclairage en couleurs (74) ;
(c) le fait de s'assurer qu'une disposition spatiale par rapport à ladite partie pendant les étapes (a) et (b) est substantiellement fixe ; et
(d) la mise en correspondance de manière conforme desdites données de couleur avec lesdites données de profondeur pour ledit réseau de référence comprenant la mise en correspondance de valeurs de couleur au niveau de chaque coordonnée X, Y des données d'image en couleurs bidimensionnelle avec des coordonnées spatiales dans les données de profondeur ayant les mêmes coordonnées X, Y pour fournir une entité numérique (I) représentant la couleur et la topologie de surface de la partie, l'entité numérique supplémentaire (I) comprenant une pluralité de points de donnée, chaque point de donnée comprenant des données de coordonnée de surface tridimensionnelle et des données de couleur mises en correspondance avec celles-ci,
dans lequel le procédé comprend en outre :
la sortie d'un rendu d'une image tridimensionnelle en pleines couleurs de la partie sur la base de l'entité numérique représentant la couleur et la topologie de surface de la partie.

30. Procédé selon la revendication 29, dans lequel dans l'étape (c), un intervalle de temps minimal est permis entre l'acquisition desdites données de profondeur et l'acquisition desdites données d'image.

31. Procédé selon la revendication 30, dans lequel ledit intervalle de temps est entre environ 0 secondes à environ 100 millisecondes, de manière préférée entre 0 et 50 millisecondes, et de manière plus préférée entre 0 et 20 millisecondes.

32. Procédé selon la revendication 29, dans lequel lesdites données de profondeur sont fournies en utilisant des techniques d'imagerie confocale.

33. Procédé selon la revendication 29, dans lequel l'étape (b) est basée sur l'un quelconque des éclairages suivants :
(A) l'éclairage de ladite partie avec trois rayonnements d'éclairage de couleurs différentes, lesdits rayonnements d'éclairage pouvant être combinés pour produire un rayonnement blanc, la capture d'une image monochrome de ladite partie correspondant à chacun desdits rayonnements d'éclairage, et la combinaison des images monochromes pour créer une image en pleines couleurs, dans lequel chacun desdits rayonnement d'éclairage est fourni sous la forme d'un deuxième réseau de faisceaux de lumière incidents (48) émis vers la partie le long d'un chemin optique à travers ladite unité de sondage pour générer des taches éclairées sur ladite partie le long de ladite direction de profondeur, dans lequel ledit deuxième réseau est défini dans les limites d'un cadre de référence
(B) l'éclairage de ladite partie avec un rayonnement d'éclairage substantiellement blanc, le passage sélectif du rayonnement réfléchi à partir de ladite partie à travers un certain nombre de filtres colorés, la capture d'une image monochrome de ladite partie correspondant à chacun desdits filtres, et la combinaison des images monochromes pour créer une image en pleines couleurs, dans lequel chaque rayonnement d'éclairage est fourni sous la forme d'un deuxième réseau de faisceaux de lumière incidents émis vers la partie le long d'un chemin optique à travers ladite unité de sondage pour générer des taches éclairées sur ladite partie le long de ladite direction de profondeur, dans lequel ledit deuxième réseau est défini dans les limites dudit cadre de référence ;
(C) l'éclairage de ladite partie avec trois rayonnements d'éclairage de couleurs différentes, la capture d'une image monochrome de ladite partie correspondant à chacun desdits rayonnements d'éclairage, et la combinaison des images monochromes pour créer une image en pleines couleurs, dans lequel chacun desdits rayonnements d'éclairage est fourni sous la forme d'un deuxième réseau de faisceaux de lumière incidents (48) émis vers la partie le long d'un chemin optique à travers ladite unité de sondage pour générer des taches éclairées sur ladite partie le long de ladite direction de profondeur, dans lequel ledit deuxième réseau est défini dans les limites dudit cadre de référence, et dans lequel lesdits rayonnements d'éclairage sont fournis par ladite première source d'éclairage.

34. Procédé selon la revendication 29, dans lequel ledit deuxième réseau est agencé pour fournir des données de couleur au niveau d'une pluralité de coordonnées spatiales correspondant substantiellement aux coordonnées spatiales dudit premier réseau.

35. Procédé selon la revendication 29, comprenant en outre au moins l'une des étapes suivantes :
(A1) l'utilisation du model virtuel tridimensionnel et en couleurs correspondant à ladite partie pour la construction d'un objet devant être fixé dans les limites de ladite structure ;
(B1) la conversion du modèle virtuel tridimensionnel et en couleurs correspondant à ladite partie en une forme pouvant être émise par le biais d'un support de communication à un destinataire.

36. Procédé selon la revendication 35, dans lequel dans l'étape (A1) ladite structure est un segment de dents avec au moins une dents qui manque ou une partie d'une dent et ledit objet est ladite au moins une dent qui manque ou la partie de la dent.

37. Procédé selon la revendication 29, comprenant la répétition des étapes (i) à (v) pour deux surfaces différentes de ladite structure pour fournir des topologies de surface de celle-ci ; et la combinaison des topologies de surface pour obtenir des données de couleur et topologiques représentatives de ladite structure.

38. Procédé selon la revendication 36, pour la reconstruction de la topologie d'une partie de dents, comprenant :
la détermination de topologies de surface d'au moins une surface buccale et une surface linguale de la partie de dents ;
la combinaison des topologies de surface pour obtenir des données représentatives d'une structure tridimensionnelle de ladite partie de dents.

39. Procédé selon la revendication 29, dans lequel le procédé comprend en outre :
la sortie des données d'image en couleurs bidimensionnelle de sorte qu'un viseur aide un utilisateur à pointer le dispositif de balayage intra-oral portatif pour le balayage tridimensionnel est fourni.
